(19) Europäisches Patentamt
European Patent Office
Office européen des brevets

(11) Veröffentlichungsnummer: **0 574 808 A1**

# (12) EUROPÄISCHE PATENTANMELDUNG

(21) Anmeldenummer: **93109190.4**

(22) Anmeldetag: **08.06.93**

(51) Int. Cl.5: **C07D 211/32**, A61K 31/445, A61K 31/495, C07D 295/10

(30) Priorität: **11.06.92 DE 4219158**

(43) Veröffentlichungstag der Anmeldung: **22.12.93 Patentblatt 93/51**

(84) Benannte Vertragsstaaten: **AT BE CH DE DK ES FR GB GR IE IT LI LU NL PT SE**

(71) Anmelder: **Dr. Karl Thomae GmbH**
**Birkendorferstrasse 65**
**D-88397 Biberach(DE)**

(72) Erfinder: **Pieper, Helmut, Dr. Dipl.-Chem.**
**Kapellenweg 5**
**D-7950 Biberach(DE)**
Erfinder: **Himmelsbach, Frank, Dr. Dipl.-Chem.**
**Ahornweg 16**
**D-7951 Mittelbiberach(DE)**
Erfinder: **Linz, Günter, Dr. Dipl.-Chem.**
**Erlenweg 8**
**D-7951 Mittelbiberach(DE)**
Erfinder: **Austel, Volkhard, Dr. Dipl.-Chem.**
**Kapellenweg 7**
**W-7950 Biberach 1(DE)**
Erfinder: **Müller, Thomas, Dr., Dipl.-Chem.**
**Alter Postplatz 17**
**W-7950 Biberach 1(DE)**
Erfinder: **Weisenberger, Johannes, Dr., Dipl.-Chem.**
**Haydnweg 5**
**W-7950 Biberach 1(DE)**

## (54) Anidine-Biphenylderivate, diese Verbindungen enthaltende Arzneimittel und Verfahren zu ihrer Herstellung.

(57) Die Erfindung betrifft Biphenylderivate der allgemeinen Formel

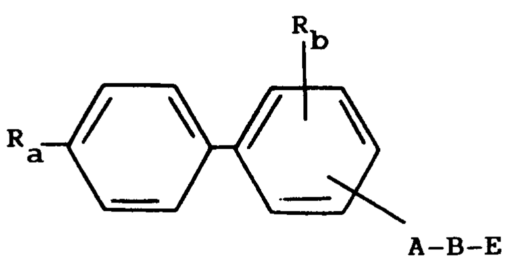

,(I)

in der
A bis E und X wie im Anspruch 1 definiert sind, deren Stereoisomere, einschließlich ihrer Gemische und deren Salze, insbesondere deren physiologisch verträgliche Salze mit anorganischen oder organischen Säuren, welche wertvolle Eigenschaften aufweisen, vorzugsweise aggregationshemmende Wirkungen, diese Verbindungen enthaltende Arzneimittel und Verfahren zu ihrer Herstellung.

EP 0 574 808 A1

Die Erfindung betrifft Biphenylderivate der allgemeinen Formel

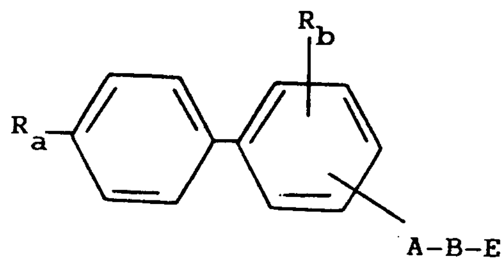

,(I)

deren Stereoisomere, einschließlich ihrer Gemische und deren Salze, insbesondere deren physiologisch verträgliche Salze mit anorganischen oder organischen Säuren, welche wertvolle Eigenschaften, insbesondere wertvolle pharmakologische Eigenschaften, aufweisen, vorzugsweise aggregationshemmende Wirkungen, diese Verbindungen enthaltende Arzneimittel, deren Verwendung und Verfahren zu ihrer Herstellung.

In der obigen allgemeinen Formel I bedeutet mit der Maßgabe, daß $R_a$ keine gegebenenfalls durch eine Alkoxycarbonylgruppe mit insgesamt 2 oder 3 Kohlenstoffatomen oder keine durch eine Benzyloxycarbonylgruppe substituierte Amidinogruppe darstellt, wenn E eine Alkoxycarbonylgruppe mit insgesamt 2 oder 3 Kohlenstoffatomen oder eine Benzyloxycarbonylgruppe darstellt,

$R_a$ eine gegebenenfalls durch eine $R_1$-CO-O-($R_2CR_3$)-O-CO-, Alkoxycarbonyl, Phenylalkoxycarbonyl-, Alkenyloxycarbonyl- oder Phenylalkenyloxycarbonylgruppe substituierte Amidinogruppe, wobei

die vorstehend erwähnten Alkoxyteile jeweils 1 bis 6 Kohlenstoffatome und der vorstehend erwähnte Alkenylteil 3 bis 5 Kohlenstoffatome enthalten kann,

$R_1$ eine geradkettige oder verzweigte Alkyl- oder Alkoxygruppe mit jeweils 1 bis 5 Kohlenstoffatomen, eine Cycloalkyl- oder Cycloalkoxygruppe mit jeweils 5 bis 7 Kohlenstoffatomen, eine Phenylalkyl-, Phenylalkoxy, Phenyl- oder Phenoxygruppe,

$R_2$ ein Wasserstoffatom, eine Alkylgruppe mit 1 bis 6 Kohlenstoffatomen, eine Cycloalkylgruppe mit 3 bis 7 Kohlenstoffatomen oder eine Phenylgruppe und

$R_3$ ein Wasserstoffatom oder eine Alkylgruppe mit 1 bis 6 Kohlenstoffatomen darstellen,

$R_b$ ein Wasserstoffatom, eine Alkyl-, Hydroxy oder Alkoxygruppe,

A eine Bindung, eine $-CH_2-$, -CO, $-CH_2CO-$ oder $-O-CH_2CO-$Gruppe, wobei die $-CH_2CO-$ und $-O-CH_2CO-$Gruppe jeweils über die Carbonylgruppe mit dem Rest B verknüpft ist, und

B eine $-NR_4-CH_2CH_2-X-(CH_2)_n-$Gruppe, in der X eine Bindung, eine $-HCR_5-$ oder $-NR_5-$Gruppe darstellt, eine $-NR_4-CH_2CH_2-R_6C=CH-$, $-NR_4-CO-(CH_2)_m-$ oder -Y-W-Gruppe, in der Y eine $NR_4$-Gruppe oder auch, wenn A eine Bindung darstellt, ein Sauerstoffatom darstellt, wobei die vorstehenden Y- oder $NR_4$-Reste jeweils mit dem Rest A verknüpft sind sowie

n die Zahl 0 oder 1,

m die Zahl 2, 3, 4 oder 5,

$R_4$ ein Wasserstoffatom, eine Alkyl- oder Phenylalkylgruppe,

$R_5$ ein Wasserstoffatom oder

$R_4$ und $R_5$ sowie $R_4$ und $R_6$ zusammen jeweils eine Ethylengruppe und

W eine geradkettige Alkylengruppe mit 2 bis 5 Kohlenstoffatomen, wobei die Ethylengruppe durch eine Phenylalkylaminocarbonylgruppe substituiert sein kann, oder eine 1,4-Cyclohexylengruppe darstellen,

E eine Alkoxycarbonylgruppe, in der der Alkoxyteil 2 bis 6 Kohlenstoffatome enthalten kann, eine Phenylalkoxycarbonylgruppe, eine Cycloalkoxycarbonylgruppe mit insgesamt 4 bis 10 Kohlenstoffatomen, in welcher der Cycloalkylteil mit 5 bis 8 Kohlenstoffatomen zusätzlich durch 1 oder 2 Alkylgruppen substituiert sein kann, eine Cycloalkoxycarbonylgruppe mit insgesamt 6 bis 8 Kohlenstoffatomen, in der im Cycloalkoxyteil eine Methylengruppe in 3- oder 4-Stellung durch ein Sauerstoffatom oder durch eine gegebenenfalls durch eine Alkyl-, Phenylalkyl- oder Phenylalkoxycarbonylgruppe oder durch eine Alkanoylgruppe mit 2 bis 6 Kohlenstoffatomen substituierte Iminogruppe ersetzt ist und der Cycloalkoxyteil zusätzlich durch 1 oder 2 Alkylgruppen substituiert sein kann, eine Cycloalkenyloxycarbonylgruppe, in der der Cycloalkenylteil 5 bis 8 Kohlenstoffatome enthalten kann, eine Alkenyloxycarbonyl-, Phenylalkenyloxycarbonyl-, Alkinyloxycarbonyl- oder Phenylalkinyloxycarbonylgruppe mit der Maßgabe, daß keine Bindung an das Sauerstoffatom von einem Kohlenstoffatom ausgeht, welches eine Doppel- oder Dreifachbindung trägt und in denen der Alkenyl- und Alkinylteil jeweils 3 bis 5 Kohlenstoffatome enthalten kann, eine Cycloalkylalkoxycarbonylgruppe, in der der Cycloalkylteil 3 bis 8 Kohlenstoffatome enthalten kann, eine Bicycloalkoxycarbonylgruppe mit insgesamt 8 bis 10 Kohlenstoffatomen, die im Bicycloalkylteil zusätzlich durch ein oder zwei Alkylgruppen

substituiert sein kann, eine $R_1$-CO-O-($R_2CR_3$)-O-CO-Gruppe, in der $R_1$ bis $R_3$ wie vorstehend erwähnt definiert sind, eine $R_7$-CO-$CH_2$-O-CO-Gruppe, in der $R_7$ eine Aminogruppe, die durch 1 oder 2 Alkylgruppen mit jeweils 1 bis 6 Kohlenstoffatomen oder durch eine Cycloalkylgruppe mit 3 bis 8 Kohlenstoffatomen substituiert sein kann, eine Pyrrolidinyl-, Piperidinyl-, Hexamethylenimino-, Morpholinyl- oder N-Alkylpiperazinylgruppe darstellt, oder eine 1,3-Dihydro-3-oxo-1-isobenzfuranyloxycarbonylgruppe,

wobei, soweit nichts anderes erwähnt wurde, die vorstehend erwähnten Alkyl- und Alkoxyteile jeweils 1 bis 3 Kohlenstoffatome enthalten können sowie

die bei der Definition der Reste $R_a$, B und E vorstehend erwähnten Phenylkerne jeweils durch Fluor-, Chlor- oder Bromatome, durch Methyl-, Ethyl-, n-Propyl-, Isopropyl-, Hydroxy-, Methoxy-, Ethoxy-, n-Propoxy-, Isopropoxy-, Trifluormethyl-, Nitro-, Amino-, Methylamino-, Ethylamino-, n-Propylamino-, Isopropylamino-, Acetamino-, Methansulfonylamino-, Carboxy-, Methoxycarbonyl-, Ethoxycarbonyl-, n-Propoxycarbonyl- oder Isopropoxycarbonylgruppe mono- oder disubstituiert sein und die Substituenten gleich oder verschieden sein können,

deren Stereoisomere, einschließlich ihrer Gemische und deren Salze.

Bevorzugte Verbindungen der obigen allgemeinen Formel I sind mit der Maßgabe, daß $R_a$ keine gegebenenfalls durch eine Alkoxycarbonylgruppe mit insgesamt 2 oder 3 Kohlenstoffatomen oder keine durch eine Benzyloxycarbonylgruppe substituierte Amidinogruppe darstellt, wenn E eine Alkoxycarbonylgruppe mit insgesamt 2 oder 3 Kohlenstoffatomen oder eine Benzyloxycarbonylgruppe darstellt, diejenigen, in denen

$R_a$ eine gegebenenfalls durch eine $R_1$-CO-O-($R_2CR_3$)-O-CO-, Alkoxycarbonyl-, Phenylalkoxycarbonyl- oder Alkenyloxycarbonylgruppe substituierte Amidinogruppe, wobei

die vorstehend erwähnten Alkoxylteile jeweils 1 bis 3 Kohlenstoffatome und der vorstehend erwähnte Alkenylteil 3 oder 4 Kohlenstoffatome enthalten kann,

$R_1$ eine geradkettige oder verzweigte Alkylgruppe mit 1 bis 5 Kohlenstoffatomen, eine Phenylalkylgruppe mit 1 bis 3 Kohlenstoffatomen im Alkylteil, eine Alkoxygruppe mit 1 bis 3 Kohlenstoffatomen, eine Cycloalkyl- oder Cycloalkoxygruppe mit jeweils 5 bis 7 Kohlenstoffatomen oder eine Phenylgruppe,

$R_2$ ein Wasserstoffatom, eine Alkylgruppe mit 1 bis 3 Kohlenstoffatomen, eine Cycloalkylgruppe mit 5 bis 7 Kohlenstoffatomen oder eine Phenylgruppe und

$R_3$ ein Wasserstoffatom darstellen,

$R_b$ ein Wasserstoffatom, eine Hydroxy- oder Alkoxygruppe mit 1 bis 3 Kohlenstoffatomen,

A eine Bindung, eine -$CH_2$-, -CO-, -$CH_2$CO- oder -O-$CH_2$CO-Gruppe, wobei die -$CH_2$CO- und -O-$CH_2$CO-Gruppe jeweils über die Carbonylgruppe mit dem Rest B verknüpft ist, und

B eine -$NR_4$-$CH_2CH_2$-X-$(CH_2)_n$-Gruppe, in der X eine Bindung, eine -$HCR_5$- oder -$NR_5$-Gruppe darstellt, eine -$NR_4$-$CH_2CH_2$-$R_6$C=CH-, -$NR_4$-CO-$(CH_2)_m$- oder -Y-W-Gruppe, in der Y eine -$NR_4$-Gruppe oder auch, wenn A eine Bindung darstellt, ein Sauerstoffatom darstellt, wobei die vorstehenden Y- oder $NR_4$-Reste jeweils mit dem Rest A verknüpft sind sowie

m die Zahl 2, 3, 4 oder 5,

n die Zahl 0 oder 1,

$R_4$ ein Wasserstoffatom, eine Alkylgruppe mit 1 bis 3 Kohlenstoffatomen oder eine Benzylgruppe,

$R_5$ ein Wasserstoffatom oder

$R_4$ und $R_5$ sowie $R_4$ und $R_6$ zusammen jeweils eine Ethylengruppe und

W eine geradkettige Alkylengruppe mit 2 bis 5 Kohlenstoffatomen, wobei die Ethylengruppe in $\alpha$-Stellung durch eine Phenylethylaminocarbonylgruppe substituiert sein kann, oder eine 1,4-Cyclohexylengruppe darstellen,

E eine Alkoxycarbonylgruppe, in der der Alkoxyteil 2 bis 6 Kohlenstoffatome enthalten kann, eine Benzyloxycarbonylgruppe, eine Cycloalkoxycarbonylgruppe mit insgesamt 4 bis 10 Kohlenstoffatomen, in welcher der Cycloalkylteil mit 5 bis 8 Kohlenstoffatomen zusätzlich durch 1 oder 2 Alkylgruppen mit jeweils 1 bis 3 Kohlenstoffatomen substituiert sein kann, eine Cycloalkoxycarbonylgruppe mit insgesamt 6 bis 8 Kohlenstoffatomen, in der im Cycloalkoxyteil eine Methylengruppe in 3- oder 4-Stellung durch ein Sauerstoffatom oder durch eine gegebenenfalls durch eine Methyl-, Benzyl- oder Benzyloxycarbonylgruppe oder durch eine Alkanoylgruppe mit 2 bis 4 Kohlenstoffatomen substituierte Iminogruppe ersetzt ist, eine Cycloalkylalkoxycarbonylgruppe, in der der Cycloalkylteil 5 bis 7 Kohlenstoffatome und der Alkoxyteil 1 oder 2 Kohlenstoffatome enthalten kann, eine Bicycloalkoxycarbonylgruppe mit insgesamt 8 oder 9 Kohlenstoffatomen, die im Bicycloalkylteil zusätzlich durch ein oder zwei Methylgruppen substituiert sein kann, eine $R_1$-CO-O-($R_2CR_3$)-O-CO-Gruppe, in der $R_1$ bis $R_3$ wie vorstehend erwähnt definiert sind, eine $R_7$-CO-$CH_2$-O-CO-Gruppe, in der $R_7$ eine Aminogruppe, die durch 1 oder 2 Alkylgruppen mit jeweils 1 bis 3 Kohlenstoffatomen oder durch eine Cycloalkylgruppe mit 5 bis 7 Kohlenstoffatomen substituiert sein kann, eine Pyrrolidinyl-, Piperidinyl-, Hexamethylenimino-, Morpholinyl- oder N-Methyl-piperazinylgruppe darstellt, oder

eine 1,3-Dihydro-3-oxo-1-isobenzfuranyloxycarbonylgruppe bedeuten,

wobei die bei der Definition der Reste $R_a$, B und E vorstehend erwähnten Phenylkerne jeweils durch Fluor-, Chlor- oder Bromatome, durch Methyl-, Hydroxy-, Methoxy-, Trifluormethyl-, Nitro-, Amino-, Methylamino-, Acetamino-, Methansulfonylamino-, Carboxy-, Methoxycarbonyl-, Ethoxycarbonyl-, n-Propoxycarbonyl- oder Isopropoxycarbonylgruppe mono- oder disubstituiert sein und die Substituenten gleich oder verschieden sein können,

insbesondere diejenigen Verbindungen, in denen

$R_a$ eine gegebenenfalls durch eine $R_1$-CO-O-($R_2CR_3$)-O-CO-, Alkoxycarbonyl, Benzyloxycarbonyl- oder Allyloxycarbonylgruppe substituierte Amidinogruppe, wobei

der vorstehend erwähnte Alkoxyteil 1 bis 3 Kohlenstoffatome enthalten kann,

$R_1$ eine geradkettige oder verzweigte Alkylgruppe mit 1 bis 4 Kohlenstoffatomen, eine Alkoxygruppe mit 1 bis 3 Kohlenstoffatomen oder eine Cyclohexyloxygruppe,

$R_2$ ein Wasserstoffatom oder eine Methylgruppe und

$R_3$ ein Wasserstoffatom darstellen,

$R_b$ ein Wasserstoffatom oder eine Methoxygruppe,

A eine Bindung, eine $-CH_2-$, -CO-, $-CH_2CO-$ oder $-O-CH_2CO$-Gruppe, wobei die $-CH_2CO-$ und $-O-CH_2CO-$Gruppe jeweils über die Carbonylgruppe mit dem Rest B verknüpft ist, und

B eine $-NR_4-CH_2CH_2-X-(CH_2)_n$-Gruppe, in der X eine Bindung, eine $-HCR_5-$ oder $-NR_5$-Gruppe darstellt, eine $-NR_4-CH_2CH_2-R_6C=CH-$, $-NR_4-CO-(CH_2)_m-$ oder -Y-W-Gruppe, in der Y eine $-NR_4$-Gruppe oder auch, wenn A eine Bindung darstellt, ein Sauerstoffatom darstellt, wobei die vor-stehenden Y- oder $NR_4$-Reste jeweils mit dem Rest A verknüpft sind sowie

m die Zahl 2, 3 oder 4,

n die Zahl 0 oder 1

$R_4$ ein Wasserstoffatom, eine Alkylgruppe mit 1 bis 3 Kohlenstoffatomen oder eine Benzylgruppe,

$R_5$ ein Wasserstoffatom oder

$R_4$ und $R_5$ sowie $R_4$ und $R_6$ zusammen jeweils eine Ethylengruppe und

W eine geradkettige Alkylengruppe mit 2 bis 5 Kohlenstoffatomen, wobei die Ethylengruppe in $\alpha$-Stellung durch eine Methoxyphenylethylaminocarbonylgruppe substituiert sein kann, oder eine 1,4-Cyclohexylengruppe darstellen,

E eine Alkoxycarbonylgruppe, in der der Alkoxyteil 2 bis 6 Kohlenstoffatome enthalten kann, eine Cycloalkoxycarbonylgruppe mit insgesamt 6 bis 10 Kohlenstoffatomen, in welcher der Cyclohexylteil zusätzlich durch 1 oder 2 Alkylgruppen mit jeweils 1 bis 3 Kohlenstoffatomen substituiert sein kann, eine Cycloalkoxycarbonylgruppe mit insgesamt 6 bis 8 Kohlenstoffatomen, in der im Cycloalkoxyteil eine Methylengruppe in 3- oder 4-Stellung durch ein Sauerstoffatom oder durch eine gegebenenfalls durch eine Methyl-, Benzyl-, Benzyloxycarbonyl- oder Acetylgruppe substituierte Iminogruppe ersetzt ist, eine Cycloalkylalkoxycarbonylgruppe, in der der Cycloalkylteil 5 oder 6 Kohlenstoffatome und der Alkoxyteil 1 oder 2 Kohlenstoffatome enthalten kann, eine Bicycloalkoxycarbonylgruppe mit insgesamt 8 Kohlenstoffatomen, eine $R_1$-CO-O-($R_2CR_3$)-O-CO-Gruppe, in der $R_1$ bis $R_3$ wie vorstehend erwähnt definiert sind, eine $R_7-CO-CH_2-O-CO$-Gruppe, in der $R_7$ eine Aminogruppe, die durch 1 oder 2 Methylgruppen oder durch eine Cyclohexylgruppe substituiert sein kann, eine Piperidinyl- oder Morpholinylgruppe darstellt, oder eine 1,3-Dihydro-3-oxo-1-isobenzfuranyloxycarbonylgruppe bedeuten,

deren Stereoisomere, einschließlich ihrer Gemische und deren Salze.

Besonders bevorzugte Verbindungen der obigen allgemeinen Formel I sind mit der Maßgabe, daß $R_a$ keine gegebenenfalls durch eine Alkoxycarbonylgruppe mit insgesamt 2 oder 3 Kohlenstoffatomen oder keine durch eine Benzyloxycarbonylgruppe substituierte Amidinogruppe darstellt, wenn E eine Alkoxycarbonylgruppe mit insgesamt 2 oder 3 Kohlenstoffatomen oder eine Benzyloxycarbonylgruppe darstellt, diejenigen, in denen

$R_a$ eine gegebenenfalls durch eine $R_1$-CO-O-($R_2CR_3$)-O-CO-, Methoxycarbonyl- oder Allyloxycarbonylgruppe substituierte Amidinogruppe, wobei

$R_1$ eine geradkettige oder verzweigte Alkylgruppe mit 1 bis 4 Kohlenstoffatomen, eine Alkoxygruppe mit 1 bis 3 Kohlenstoffatomen oder eine Cyclohexyloxygruppe,

$R_2$ ein Wasserstoffatom oder eine Methylgruppe und

$R_3$ ein Wasserstoffatom darstellen,

$R_b$ ein Wasserstoffatom oder eine Methoxygruppe,

A eine $-CH_2-$ oder -CO-Gruppe und

B eine $-NR_4-CH_2CH_2-X-CH_2$-Gruppe, in der X eine $-NR_5$-Gruppe darstellt, eine $-NR_4-CH_2CH_2-R_6C=CH-$ oder -Y-W-Gruppe, in der Y eine $-NR_4$-Gruppe darstellt, wobei die vorstehenden Y- oder $NR_4$-Reste jeweils mit dem Rest A verknüpft sind sowie

$R_4$ ein Wasserstoffatom oder eine Methylgruppe oder
$R_4$ und $R_5$ sowie $R_4$ und $R_6$ zusammen jeweils eine Ethylengruppe und
W eine 1,4-Cyclohexylengruppe darstellen,

E eine Isopropyloxycarbonyl-, Isobutyloxycarbonyl-, 2-Butyloxycarbonyl-, 2-Amyloxycarbonyl-, 3-Amyloxycarbonyl- oder Neopentyloxycarbonylgruppe, eine Cycloalkoxycarbonylgruppe mit insgesamt 6 bis 9 Kohlenstoffatomen, in welcher der Cyclohexylteil zusätzlich durch 1 oder 2 Alkylgruppen mit jeweils 1 bis 3 Kohlenstoffatomen substituiert sein kann, eine Cycloalkylalkoxycarbonylgruppe, in der der Cycloalkylteil 5 oder 6 Kohlenstoffatome und der Alkoxyteil 1 oder 2 Kohlenstoffatome enthalten kann, eine Bicycloalkoxycarbonylgruppe mit insgesamt 8 Kohlenstoffatomen, eine $R_1$-CO-O-($R_2CR_3$)-O-CO-Gruppe, in der $R_1$ bis $R_3$ wie vorstehend erwähnt definiert sind, bedeuten,

deren Stereoisomere, einschließlich ihrer Gemische und deren Salze, insbesondere jedoch die Verbindungen

4-Amidino-4'-[4-(cyclohexyloxycarbonylmethyl)-piperidinocarbonyl]-biphenyl,
4-Amidino-4'-[4-(cyclopentyloxycarbonylmethyl)-piperidinocarbonyl]-biphenyl,
4-Amidino-3'-[4-trans-(cyclohexyloxycarbonyl)-cyclohexylaminocarbonyl]-4'-methoxy-biphenyl,
4-Amidino-3'-[4-trans-(cyclohexylmethoxycarbonyl)-cyclohexylaminocarbonyl]-4'-methoxy-biphenyl,
4-Amidino-4'-[4-(cycloheptyloxycarbonylmethyl)-piperidinocarbonyl]-biphenyl,
4-Amidino-4'-[4-((exo)-norbornyloxycarbonylmethyl)-piperidinocarbonyl]-biphenyl,
4-Amidino-4'-[4-((-)-menthyloxycarbonylmethyl)-piperidinocarbonyl]-biphenyl,
4-Amidino-4'-[4-(pivaloyloxymethyloxycarbonylmethyl)-piperidinocarbonyl]-biphenyl,
4-[4-[(1-(Äthoxycarbonyloxy)-äthoxycarbonyl)-methyl]-piperidinocarbonyl]-4'-amidino-biphenyl,
[4-(N-Allyloxycarbonyl-amidino]-4'-[4-(methoxycarbonylmethyl)-piperidinocarbonyl]-biphenyl,
4-[N-Allyloxycarbonyl-amidino]-4'-[4-(carboxymethyl)-piperidinocarbonyl]-biphenyl und
4-[4-(Cyclohexyloxycarbonylmethyl)-piperidinocarbonyl]-4'-[N-methoxycarbonyl-amidino]-biphenyl,
deren Stereoisomere, einschließlich ihrer Gemische und deren Salze.

Erfindungsgemäß erhält man die neuen Verbindungen beispielsweise nach folgenden Verfahren:

a) Zur Herstellung von Verbindungen der allgemeinen Formel I, in der E eine Alkoxycarbonylgruppe mit insgesamt 3 bis 7 Kohlenstoffatomen, eine Phenylalkoxycarbonylgruppe, eine Cycloalkoxycarbonylgruppe mit insgesamt 4 bis 10 Kohlenstoffatomen, in welcher der Cycloalkylteil mit 5 bis 8 Kohlenstoffatomen zusätzlich durch 1 oder 2 Alkylgruppen substituiert sein kann, eine Cycloalkoxycarbonylgruppe mit insgesamt 6 bis 8 Kohlenstoffatomen, in der im Cycloalkoxyteil eine Methylengruppe in 3- oder 4-Stellung durch ein Sauerstoffatom oder durch eine gegebenenfalls durch eine Alkyl-, Phenylalkyl- oder Phenylalkoxycarbonylgruppe oder durch eine Alkanoylgruppe mit 2 bis 6 Kohlenstoffatomen substituierte Iminogruppe ersetzt ist und der Cycloalkoxyteil zusätzlich durch 1 oder 2 Alkylgruppen substituiert sein kann, eine Cycloalkenyloxycarbonylgruppe, in der der Cycloalkenylteil 5 bis 8 Kohlenstoffatome enthalten kann, eine Alkenyloxycarbonyl-, Phenylalkenyloxycarbonyl-, Alkinyloxycarbonyl- oder Phenylalkinyloxycarbonylgruppe mit der Maßgabe, daß keine Bindung an das Sauerstoffatom von einem Kohlenstoffatom ausgeht, welches eine Doppel- oder Dreifachbindung tragt und in denen der Alkenyl- und Alkinylteil jeweils 3 bis 5 Kohlenstoffatome enthalten kann, eine Cycloalkylalkoxycarbonylgruppe, in der der Cycloalkylteil 3 bis 8 Kohlenstoffatome enthalten kann, oder eine Bicycloalkoxycarbonylgruppe mit insgesamt 8 bis 10 Kohlenstoffatomen, die im Bicycloalkylteil zusätzlich durch ein oder zwei Alkylgruppen substituiert sein kann, darstellt:

Veresterung einer Carbonsäure der allgemeinen Formel

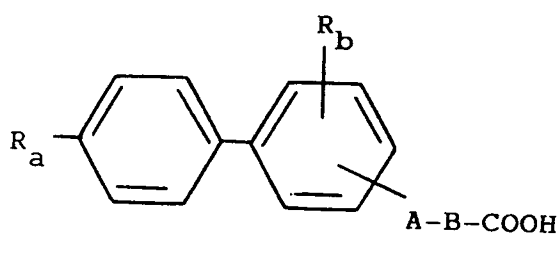

,(II)

in der

$R_a$, $R_b$, A und B wie eingangs definiert sind, oder deren gegebenenfalls im Reaktionsgemisch gebildeten reaktionsfähigen Derivaten, mit einem Alkohol der allgemeinen Formel

HO - $R_8$ ,(III)

in der

$R_8$ eine Alkylgruppe mit 2 bis 6 Kohlenstoffatomen, eine Phenylalkylgruppe, eine Cycloalkylgruppe mit 3 bis 9 Kohlenstoffatomen, in welcher der Cycloalkylteil mit 5 bis 8 Kohlenstoffatomen zusätzlich durch 1 oder 2 Alkylgruppen substituiert sein kann, eine Cycloalkylgruppe mit 5 bis 7 Kohlenstoffatomen, in der im Cycloalkylteil eine Methylengruppe in 3- oder 4-Stellung durch ein Sauerstoffatom oder durch eine gegebenenfalls durch eine Alkyl-, Phenylalkyl- oder Phenylalkoxycarbonylgruppe oder durch eine Alkanoylgruppe mit 2 bis 6 Kohlenstoffatomen substituierte Iminogruppe ersetzt ist und der Cycloalkylteil zusätzlich durch 1 oder 2 Alkylgruppen substituiert sein kann, eine Cycloalkenylgruppe, in der der Cycloalkenylteil 5 bis 8 Kohlenstoffatome enthalten kann, eine Alkenyl-, Phenylalkenyl-, Alkinyl- oder Phenylalkinylgruppe mit der Maßgabe, daß keine Bindung an das Sauerstoffmolekül von einem Kohlenstoffatom ausgeht, welches eine Doppel- oder Dreifachbindung trägt und in denen der Alkenyl- und Alkinylteil jeweils 3 bis 5 Kohlenstoffatome enthalten kann, eine Cycloalkylalkylgruppe, in der der Cycloalkylteil 3 bis 8 Kohlenstoffatome enthalten kann, oder eine Bicycloalkylgruppe mit insgesamt 7 bis 9 Kohlenstoffatomen, die im Bicycloalkylteil zusätzlich durch ein oder zwei Alkylgruppen substituiert sein kann, darstellt.

Die Veresterung wird zweckmäßigerweise in einem Lösungsmittel oder Lösungsmittelgemisch wie Methylenchlorid, Dimethylformamid, Dimethylsulfoxid, Toluol, Chlorbenzol, Tetrahydrofuran, Toluol/Tetrahydrofuran oder Dioxan gegebenenfalls in Gegenwart einer Säure wie Salzsäure oder in Gegenwart eines wasserentziehenden Mittels, z.B. in Gegenwart von einem Chlorameisensäureester wie Chlorameisensäureisobutylester, Thionylchlorid, Trimethylchlorsilan, Titantetrachlorid, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol, N,N'-Carbonyldiimidazol- oder N,N'-Thionyldiimidazol gegebenenfalls in Gegenwart einer Base wie eines in 4-Stellung durch eine sekundäre Aminogruppe substituierten Pyridins wie 4-Dimethylamino-pyridin oder Triphenylphosphin/Tetrachlorkohlenstoffbei Temperaturen zwischen -30 und 100 °C, vorzugsweise jedoch bei Temperaturen zwischen -10 und 60 °C, durchgeführt.

b) Umsetzung einer Carbonsäure der allgemeinen Formel

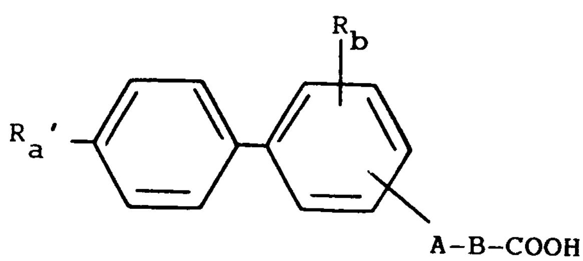

,(IV)

in der

$R_b$, A und B wie eingangs definiert sind und

$R_a'$ eine durch eine $R_1$-CO-O-($R_2CR_3$)-O-CO-, Alkoxycarbonyl-, Phenylalkoxycarbonyl-, Alkenyloxycarbonyl- oder Phenylalkenyloxycarbonylgruppe substituierte Amidinogruppe, wobei der vorstehend erwähnte Alkoxylteil jeweils 1 bis 6 Kohlenstoffatomen und der vorstehend erwähnte Alkenylteil 3 bis 5 Kohlenstoffatome enthalten kann, oder eine durch einen Schutzrest geschützte Amidinogruppe darstellt, oder dessen Carbanion mit einer Verbindung der allgemeinen Formel

$Z_1$ - $R_9$ ,(V)

in der

$R_9$ eine Alkylgruppe mit 2 bis 6 Kohlenstoffatomen, eine Phenylalkylgruppe, eine Cycloalkylgruppe mit 3 bis 9 Kohlenstoffatomen, in welcher der Cycloalkylteil mit 5 bis 8 Kohlenstoffatomen zusätzlich durch 1 oder 2 Alkylgruppen substituiert sein kann, eine Cycloalkylgruppe mit 5 bis 7 Kohlenstoffatomen, in der im Cycloalkylteil eine Methylengruppe in 3- oder 4-Stellung durch ein Sauerstoffatom oder durch eine gegebenenfalls durch eine Alkyl-, Phenylalkyl- oder Phenyloxycarbonylgruppe oder durch eine Alkanoylgruppe mit 2 bis 6 Kohlenstoffatomen substituierte Iminogruppe ersetzt ist und der Cycloalkylteil zusätzlich durch 1 oder 2 Alkylgruppen substituiert sein kann, eine Cycloalkenylgruppe mit 5 bis 8 Kohlenstoffatomen, eine Alkenyl-, Phenylalkenyl-, Alkinyl- oder Phenylalkinylgruppe mit der Maßgabe,

daß keine Bindung an $Z_1$ von einem Kohlenstoffatom ausgeht, welches eine Doppel- oder Dreifachbindung trägt und in denen der Alkenyl- und Alkinylteil jeweils 3 bis 5 Kohlenstoffatome enthalten kann, eine Cycloalkylalkylgruppe, in der der Cycloalkylteil 3 bis 8 Kohlenstoffatome enthalten kann, oder eine Bicycloalkylgruppe mit insgesamt 7 bis 9 Kohlenstoffatomen, die im Bicycloalkylteil zusätzlich durch ein oder zwei Alkylgruppen substituiert sein kann, eine $R_1$-CO-O-($R_2CR_3$)-Gruppe, in der $R_1$ bis $R_3$ wie vorstehend erwähnt definiert sind, eine $R_7$-CO-$CH_2$-Gruppe, in der $R_7$ eine Aminogruppe, die durch 1 oder 2 Alkylgruppen mit jeweils 1 bis 6 Kohlenstoffatomen oder durch eine Cycloalkylgruppe mit 3 bis 8 Kohlenstoffatomen substituiert sein kann, eine Pyrrolidinyl-, Piperidinyl-, Hexamethylenimino-, Morpholinyl- oder N-Alkylpiperazinyl-gruppe darstellt, oder eine 1,3-Dihydro-3-oxo-1-isobenzfuranylgruppe und

$Z_1$ eine nukleophile Austrittsgruppe wie ein Halogenatom, z. B. ein Chlor- oder Bromatom, darstellen und erforderlichenfalls anschließende Abspaltung des verwendeten Schutzrestes.

Als Schutzreste für die Amidinogruppen kommen beispielsweise die tert.Butyloxycarbonyl-, Allyloxycarbonyl- oder 9-Fluorenylmethyloxycarbonyl-Gruppe und

für die Hydroxygruppe beispielsweise die Trimethylsilyl-, Tetrahydropyran-2-yl-, Benzyl-, Benzhydryl- oder Triphenylmethylgruppe in Betracht.

Die Umsetzung wird zweckmäßigerweise in einem Lösungsmittel wie Methylenchlorid, Tetrahydrofuran, Dioxan, Dimethylsulfoxid oder Dimethylformamid gegebenenfalls in Gegenwart eines Reaktionsbeschleunigers wie Natrium- oder Kaliumjodid und vorzugsweise in Gegenwart einer Base wie Natriumcarbonat, Kaliumcarbonat oder Natronlauge oder in Gegenwart einer tertiären organischen Base wie N-Ethyl-diisopropylamin, N-Methyl-morpholin, Pyridin oder 4-Diemthylamino-pyridin, welche gleichzeitig auch als Lösungsmittel dienen können, oder gegebenenfalls in Gegenwart von Silberkarbonat oder Silberoxid bei Temperaturen zwischen -30 und 100 °C, vorzugsweise jedoch bei Temperaturen zwischen -10 und 80 °C, durchgeführt.

Die Abspaltung eines verwendeten Schutzrestes für die Amino-, Amidino- oder Hydroxygruppe erfolgt je nach dem verwendeten Schutzrest und je nach zu schützender Gruppe vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und bei einem Wasserstoffdruck von 1 bis 7 bar, vorzugsweise jedoch von 3 bis 5 bar, mit Hilfe einer wässrigen Mineralsäure wie Chlorwasserstoffsäure, Bromwasserstoffsäure oder Schwefelsäure, mit Hilfe einer organischen Säure wie Ameisensäure oder Trifluoressigsäure in einem Lösungsmittel wie Methanol, Ethanol, Essigsäureethylester, Methylenchlorid oder Eisessig, mit Hilfe eines sekundären Amins wie Piperidin oder Morpholin in einem Lösungsmittel wie Dimethylformamid oder Methylenchlorid oder mit Hilfe eines Katalysators wie Tetrakis-(triphenylphosphin)-palladium(0) und Morpholin in einem Lösungsmittel wie Tetrahydrofuran oder Dioxan jeweils bei Temperaturen zwischen 0 und 50 °C, vorzugsweise jedoch bei Raumtemperatur.

c) Zur Herstellung von Verbindungen der allgemeinen Formel I, in der $R_a$ eine durch eine $R_1$-CO-O-($R_2CR_3$)-O-CO-, Alkoxycarbonyl-, Alkenyloxycarbonyl-, Phenylalkoxycarbonyl- oder Phenylalkenyloxycarbonylgruppe substituierte Amidinogruppe darstellt:

Acylierung einer Verbindung der allgemeinen Formel

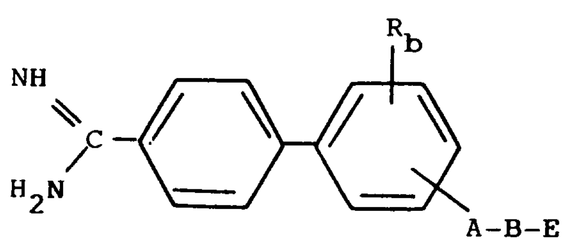

,(VI)

in der

$R_b$, A, B und E wie eingangs definiert sind, mit einer Verbindung der allgemeinen Formel

$Z_2$ - $R_{10}$ ,(VII)

in der

$R_{10}$ eine $R_1$-CO-O-($R_2CR_3$)-O-CO-, Alkoxycarbonyl-, Alkenyloxycarbonyl-, Phenylalkoxycarbonyl- oder

Phenylalkenyloxycarbonylgruppe und

$Z_2$ eine nukleophile Austrittsgruppe wie ein Halogenatom, z. B. ein Chlor- oder Bromatom oder eine p-Nitrophenylgruppe, darstellen.

Die Umsetzung wird zweckmäßigerweise in einem Lösungsmittel wie Tetrahydrofuran, Methylenchlorid, Chloroform oder Dimethylformamid zweckmäßigerweise in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100°C, vorzugsweise jedoch bei Temperaturen zwischen -10 und 80°C, durchgeführt.

Bei den vorstehend beschriebenen Umsetzungen können gegebenenfalls vorhandene reaktive Gruppen wie Hydroxy-, Carboxy-, Amino-, Alkylamino-, Imino- oder Amidinogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,
als Schutzrest für eine Carboxylgruppe die Trimethylsilyl-, Methyl-, Ethyl-, tert.Butyl- oder Benzylgruppe und

als Schutzrest für eine Amino-, Alkylamino- oder Iminogruppe die Acetyl-, tert.Butoxycarbonyl-, Benzyloxycarbonyl-, Benzyl-, Benzhydryl-, Trityl-, Methoxybenzyl-, 2,4-Dimethoxybenzyl-, 9-Fluorenylmethyloxycarbonyl- oder Allyloxycarbonylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen -10 und 100°C, vorzugsweise bei Temperaturen zwischen 0 und 60°C.

Die Abspaltung eines Benzyl-, Methoxybenzyl, Benzyloxycarbonyl-, Benzhydryl- oder Tritylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer-(IV)ammoniumnitrat in einem Lösungsmittel wie Methylenchlorid, Acetonitril oder Acetonitril/wasser bei Temperaturen zwischen 0 und 50°C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung einer 9-Fluorenylmethyloxycarbonylgruppe erfolgt vorzugsweise mit Hilfe eines sekundären Amins wie Piperidin oder Morpholin in einem Lösungsmittel wie Dimethylformamid oder Methylenchlorid bei Temperaturen zwischen 0 und 50°C, vorzugsweise jedoch bei Raumtemperatur, und die Abspaltung einer Allyloxycarbonylgruppe vorzugsweise in Gegenwart eines Katalysators wie Tetrakis-(triphenylphosphin)-palladium(0) und von Morpholin in einem Lösungsmittel wie Tetrahydrofuran oder Dioxan bei Temperaturen zwischen 0 und 50°C, vorzugsweise jedoch bei Raumtemperatur.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.- So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 asymmetrischen Kohlenstoffatomen auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der

racemischen Verbindung Salze oder Derivate wie z.B. Ester oder Amide bildenden optisch aktiven Substanz, insbesondere Säuren und ihren aktivierten Derivaten oder Alkoholen, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Essigsäure, Fumarsäure, Bernsteinsäure, Milchsäure, Zitronensäure, Weinsäure oder Maleinsäure in Betracht.

Die als Ausgangsstoffe verwendeten Verbindungen sind teilweise literaturbekannt oder man erhält diese nach literaturbekannten Verfahren. Außerdem werden diese teilweise in der nicht vorveröffentlichten EP-A-0,496,378 beschrieben. So erhält man beispielsweise eine Verbindung der allgemeinen Formel II und IV durch Acylierung eines entsprechenden Amidins, wobei das hierfür erforderliche Amidin zweckmäßigerweise durch Umsetzung eines entsprechenden Iminoesters mit einem entsprechenden Amin hergestellt wird. Eine so erhaltene Verbindung der Formel II wird anschließend mittels Hydrolyse in eine entsprechende Verbindung der allgemeinen Formel VI übergeführt.

Wie bereits eingangs erwähnt, weisen die neuen Biphenylderivate der allgemeinen Formel I und deren Salze, insbesondere deren physiologisch verträgliche Salze mit anorganischen oder organischen Säuren, wertvolle Eigenschaften auf. So weisen die neuen Verbindungen der allgemeinen Formel I in vivo wertvolle pharmakologische Eigenschaften auf, neben einer entzündungshemmenden und den Knochenabbau hemmenden Wirkung insbesondere antithrombotische, antiaggregatorische und tumor- bzw. metastasenhemmende Wirkungen.

Beispielsweise wurden die Verbindungen der allgemeinen Formel I auf ihre biologischen Wirkungen wie folgt untersucht:
Die Hemmung der Thrombozytenaggregation nach oraler Gabe der Prüfsubstanz wird ex vivo an Rhesusaffen ermittelt.

Direkt vor der oralen Verabreichung der in Natrosol suspendierten Prüfsubstanz wird den Tieren eine Blutprobe aus der Kubitalvene als Referenzwert entnommen. Zu definierten Zeiten nach der Substanzgabe werden erneut Blutproben gewonnen und wie folgt untersucht.

Das mit 3,14% Natriumcitrat im Volumenverhältnis 1:10 versetzte Vollblut wird mit 200 g für 15 Minuten zentrifugiert. Der Überstand an plättchenreichem Plasma wird vorsichtig abgehoben. Aus dem erythrozytenreichen Sediment wird durch Zentrifugation mit 4000 g für 10 Minuten das plättchenarme Plasma als Überstand gewonnen.

Die mit Collagen (Hormonchemie, München; 2 µg/ml Endkonzentration im plättchenreichen Plasma) ausgelöste Thrombozytenaggregation in diesen ex vivo Proben wird nach der Methode von Born und Cross (J. Physiol. 170, 397 (1964)) photometrisch gemessen. Die nach Collagenstimulation gemessene größte Lichtdurchlässigkeit des plättchenreichen Plasmas wird mit dem Referenzwert verglichen, um die Hemmung der Aggregation zu den verschiedenen Zeitpunkten der Blutentnahme nach Substanzgabe relativ zum Referenzwert zu bestimmen.

Die Verbindung des Beispiels 1 hemmt die Collagen-induzierte Thrombozytenaggregation ex vivo nach oraler Gabe von 3 mg/kg länger als 4 Stunden.

Die erfindungsgemäße Verbindung ist gut verträglich, da bei den applizierten Dosen keine toxischen Nebenwirkungen beobachtet werden konnten.

Auf Grund ihrer Hemmwirkung auf Zell-Zell- bzw. Zell-Matrix-Wechselwirkungen eignen sich die neuen Biphenylderivate der allgemeinen Formel I und ihre physiologisch verträglichen Additionssalze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarktes, der Arteriosklerose, der Osteoporose und der Metastasierung von Tumoren und der Therapie genetisch bedingter oder auch erworbener Störungen der Interaktionen von Zellen untereinander oder mit soliden Strukturen. Weiterhin eignen sich diese zur Begleittherapie bei der Thrombolyse mit Fibrinolytica oder Gefäßinterventionen wie transluminaler Angioplastie oder auch bei der Therapie von Schockzuständen, der Psoriasis des Diabetes und von Entzündungen.

Für die Bekämpfung bzw. Verhütung der vorstehend erwähnten Krankheiten liegt die Dosis zwischen 0,1 µg und 30 mg/kg Körpergewicht, vorzugsweise bei 1 µg bis 15 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromboxan-Rezeptor-Antagonisten und Thromboxansynthesehemmer oder deren Kombinationen, Serotonin-Antagonisten, α-Rezeptorantagonisten, Alkylnitrate wie Glycerintrinitrat, Phosphodiesterasehemmer, Prostacyclin und deren Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Stearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Sprays oder Zäpfchen einarbeiten.

Die nachfolgenden Beispiele sollen die Erfindung näher erläutern:

## Beispiel I

### 4'-Cyano-biphenylyl-4-essigsäure

Eine Mischung aus 11,3 g 4'-Brom-biphenylyl-4-essigsäure (Schmelzpunkt: 172-175 °C, hergestellt aus 4-Acetyl-4'-brombiphenyl durch Behandeln mit Morpholin und Schwefel und anschließende Hydrolyse mit Kaliumhydroxid), 3,48 g Kupfer-(I)-cyanid und 100 ml Dimethylformamid wird 12 Stunden zum Rückfluß erhitzt und nach dem Abkühlen eingeengt. Der Rückstand wird zwischen 1n Natronlauge und Methylenchlorid, dem etwas Methanol zugesetzt wird, verteilt. Die wäßrige Phase wird angesäuert und mit Methylenchlorid extrahiert. Die Methylenchloridphase wird mit Aktivkohle behandelt, eingeengt, der feste Rückstand mit einer Mischung aus Ether und Petrolether verrieben und abfiltriert.
Ausbeute: 5,1 g (55 % der Theorie),
$R_f$-Wert: 0,46 (Kieselgel; Methylenchlorid/Ethanol = 9:1)
Analog wird folgende Verbindung erhalten:
(1) 4-Cyano-biphenylyl-4'-carbonsäure-methylester
Schmelzpunkt: 140-142 °C
Die erforderliche Ausgangsverbindung 4-Brom-4'-biphenylyl-carbonsäure-methylester (Schmelzpunkt: 140-142 °C) erhält man durch Veresterung der Säure mit methanolischer Salzsäure. 4-Brom-4'-biphenylyl-carbonsäure erhält man durch Umsetzung von 4-Acetyl-4'-brom-biphenyl mit Brom und Natronlauge.

## Beispiel II

### 4-Cyano-4'-hydroxymethyl-biphenyl

Eine Lösung von 5 g (0,0224 Mol) 4'-Cyano-biphenyl-4-carbonsäure und 2,27 g (0,0224 Mol) Triethylamin in 100 ml Tetrahydrofuran wird bei 5 °C unter Rühren innerhalb 5 Minuten mit einer Lösung von 2,43 g (0,024 Mol) Chlorameisensäureäthylester in 20 ml Tetrahydrofuran versetzt. Anschließend rührt man bei 5 °C eine weitere Stunde. Das ausgefallene Triethylamin-hydrochlorid wird abgesaugt und die Mutterlauge unter Rühren bei 10-15 °C in eine Lösung von 2,12 g (0,056 Mol) Natriumborhydrid in 20 ml Wasser eingetropft. Nach weiteren 4 Stunden Rühren bei Raumtemperatur säuert man mit 2n Salzsäure bis pH 2-3 an, trennt die entstandene wäßrige Phase ab und extrahiert sie mit Essigsäureethylester. Die organischen Phasen werden vereinigt, mit 10%iger Natronlauge und anschließend mit Wasser gewaschen. Nach dem Trocknen über Natriumsulfat dampft man unter vermindertem Druck zur Trockne ein.
Ausbeute: 2,5 g (53,3 % der Theorie) eines amorphen Produktes.

## Beispiel III

### 4-Chlormethyl-4'-cyano-biphenyl

2,5 g (0,0119 Mol) 4-Cyano-4'-hydroxymethyl-biphenyl werden in 70 ml Dichlormethan gelöst und nach Zugabe von 2,4 g (0,0239 Mol) Triethylamin langsam unter Rühren bei Raumtemperatur mit 2,7 g (0,024 Mol) Mesylchlorid versetzt. Nach Stehen über Nacht wird die klare Lösung unter vermindertem Druck zur

Trockne eingedampft und der Rückstand über Kieselgel chromatographiert, wobei Methylenchlord als Elutionsmittel dient.
Ausbeute: 2,6 g (96 % eines amorphen Festkörpers)

Beispiel IV

4-Cyano-4'-iodmethyl-biphenyl

2,4 g (0,0087 Mol) 4-Chlormethyl-4'-cyano-biphenyl werden zusammen mit 6,26 g (0,042 Mol) Natriumiodid in 100 ml Aceton während 3 Stunden auf Rückflußtemperatur erhitzt. Danach engt man die Suspension im Vakuum zur Trockne ein und extrahiert den Rückstand mehrfach mit Dichlormethan. Die vereinigten Dichlormethan-Lösungen werden zur Trockne eingedampft. Der so gewonnene rohe Rückstand wird ohne weitere Reinigung in Beispiel V verwendet.

Beispiel V

4-Cyano-4'-[4-(methoxycarbonylmethyl)-piperidinomethyl]-biphenyl

Das in Beispiel V gewonnene Rohprodukt wird in 100 ml Chloroform gelöst. Diese Lösung wird nach Versetzen mit 2,5 g Piperidinoessigsäuremethylester-Hydrochlorid und 2,57 g Triethylamin während 4 Stunden auf Rückflußtemperatur erhitzt. Nach dem Erkalten wird das ausgeschiedene Triethylamin-Hydrochlorid abgesaugt und die Mutterlauge zur Trockne eingeengt. Der Rückstand wird über Kieselgel chromatographiert, wobei Dichlormethan/Methanol (40:1) als Elutionsmittel dient. Ausbeute: 2,2 g (59,6 % der Theorie)

Beispiel VI

4-Amidino-4'-[4-(methoxycarbonylmethyl)-piperidinomethyl]-biphenyl

In eine Suspension von 2,1 g (0,006 Mol) 4-Cyano-4'-[4-(methoxycarbonylmethyl)-piperidinomethyl]-biphenyl in 100 ml absolutem Methanol wird unter Eiskühlung und Rühren während 2 Stunden HCl-Gas eingeleitet. Anschließend läßt man die Reaktionsmischung 4 Stunden lang bei Raumtemperatur stehen. Die Lösung wird im Vakuum zur Trockne eingeengt, der Rückstand in Methanol gelöst und die so erhaltene Lösung bis zur basischen Reaktion mit Natriumcarbonat versetzt. Die so entstandene Suspension wird im Vakuum zur Trockne eingeengt. Der Rückstand wird über Kieselgel chromatographiert, wobei Dichlormethan/Methanol/konz.Ammoniak im Verhältnis 9:1:0,1 als Elutionsmittel dient.
Ausbeute: 0,72 g (29,7 % der Theorie),
Schmelzpunkt: 148-150 ° C

Beispiel VII

4-Amidino-4'-[4-(hydroxycarbonylmethyl)-piperidinomethyl]-biphenyl

0,59 g 4-Amidino-4'-[4-(methoxycarbonylmethyl)-piperidinomethyl]-biphenylwerden in 15 ml Tetrahydrofuran und 11 ml Wasser gelöst. In diese Lösung trägt man unter Rühren 8,1 ml einer 1n wässrigen Lithiumhydroxid-Lösung ein. Nach weiteren 3 Stunden versetzt man mit 433 mg Ammoniumchlorid, wobei sich eine farblose Substanz abscheidet. Das Tetrahydrofuran wird unter Vakuum abdestilliert und die ausgefallene Substanz anschließend abfiltriert.
Ausbeute: 420 mg (74,7 % der Theorie),
Schmelzpunkt: 318-320 ° C (Zers.)

Beispiel VIII

3-Carboxy-4'-cyano-4-hydroxy-biphenyl

Zu einer 30 Minuten bei -20 ° C gerührten Mischung aus 25 ml Oxalylchlorid, 50 ml Methylenchlorid und 25 g Aluminiumchlorid wird eine Lösung von 12 g 4-Cyano-4'-methoxy-biphenyl in 75 ml Methylenchlorid getropft. Man rührt 5 Stunden bei -20 ° C, läßt unter anfänglicher Eiskühlung im Verlauf von 16 Stunden auf

Raumtemperatur kommen und rührt dann noch weitere 3 Stunden. Man gießt auf Eiswasser, rührt 30 Minuten nach, extrahiert mit Essigester und dampft die Essigesterphase bis zur Kristallisation ein.
Ausbeute: 10,3 g (75 % der Theorie),
Schmelzpunkt: 244-246 °C

Beispiel IX

4-(tert.Butyloxycarbonylmethyloxy)-4'-cyano-biphenyl

Hergestellt analog Beispiel 13 aus 4-Cyano-4'-hydroxy-biphenyl und Bromessigsäure-tert.butylester.
Schmelzpunkt: 110-112 °C

Beispiel X

4-(Carboxymethyloxy)-4'-cyano-biphenyl

32,9 g 4-(tert.Butyloxycarbonylmethyloxy)-4'-cyano-biphenyl werden in 250 ml Methylenchlorid gelöst und langsam mit 137 ml Trifluoressigsäure versetzt. Man rührt 3 Stunden bei Raumtemperatur, dampft zur Trockene ein und verreibt den Rückstand mit Wasser.
Ausbeute: 26,3 g (98 % der Theorie),
Schmelzpunkt: 202-204 °C

Beispiel XI

4-Cyano-4'-[[[3-(2-methoxycarbonyl-ethyl)-phenyl]-aminocarbonyl]-methyl]-biphenyl

Zu einer Mischung aus 1 g 4'-Cyano-biphenylyl-4-essigsäure, 1,2 g 3-(3-Aminophenyl)-propionsäure-methylester, 0,57 g 1-Hydroxy-1H-benztriazol-hydrat und 100 ml Tetrahydrofuran wird unter Eiskühlung eine Lösung von 1,4 g Dicyclohexylcarbodiimid in 20 ml Tetrahydrofuran getropft. Man rührt noch eine Stunde weiter, läßt auf Raumtemperatur kommen und filtriert den ausgefallenen Dicyclohexylharnstoff ab. Das Filtrat wird eingeengt und der Rückstand durch Chromatographie an Kieselgel (Elutionsmittel: Methylenchlorid/Methanol = 30:1) gereinigt.
Ausbeute: 1,58 g (95 % der Theorie),
Schmelzpunkt: 166-168 °C

Beispiel XII

4-Cyano-4'-[(2-ethoxycarbonyl-ethylamino)-carbonyl]-biphenyl

Eine Mischung aus 2 g 4'-Cyano-biphenylyl-4-essigsäure, 1,9 ml N-Methyl-morpholin und 100 ml Tetrahydrofuran wird auf -30 °C abgekühlt und mit 1,1 ml Chlorameisensäure-isobutylester versetzt. Man rührt eine Stunde, fügt 1,3 g $\beta$-Alaninethylester-hydrochlorid zu und rührt 50 Stunden bei Raumtemperatur weiter. Die erhaltene Lösung wird in 300 ml 0,5 molare Kaliumhydrogensulfatlösung eingerührt und mit Essigester extrahiert. Die Essigesterphase wird eingeengt und mit Ether versetzt, wobei das Produkt kristallin anfällt.
Ausbeute: 1,1 g (39 % der Theorie),
Schmelzpunkt: 132-136 °C

Beispiel XIII

[4-Amidino-4'-(5-methoxycarbonyl-pentyloxy)-biphenyl]-dihydrogencarbonat

Man überschichtet 75 ml Methanol mit 30 ml Petroläther und leitet unter Eiskühlung Chlorwasserstoffgas bis zur Sättigung ein. Man trägt nun 2,1 g 4-Cyano-4'-(5-ethoxycarbonylpentyloxy)-biphenyl ein und rührt 18 Stunden bei Raumtemperatur. Man engt im Vakuum zur Trockene ein, suspendiert den Rückstand in Methanol, setzt 5,36 g Ammoniumcarbonat zu und rührt 16 Stunden bei Raumtemperatur. Der erhaltene Niederschlag wird abfiltriert und durch Verrühren mit Methylenchlorid/Methanol (85:15) und Wasser gereinigt.

Ausbeute: 1,75 g (75 % der Theorie),
Schmelzpunkt: 185-189 °C (Zers.)

Beispiel 1

4-Amidino-4'-[4-(cyclohexyloxycarbonylmethyl)-piperidinocarbonyl]-biphenyl-hydrochlorid

Man leitet in eine Suspension von 1 g (0,0027 Mol) 4-Amidino-4'-[4-(carboxymethyl)-piperidinocarbonyl]-biphenyl in 20 ml Dichlormethan und 20 ml Cyclohexanol bei Raumtemperatur Salzsäuregas bis zur Sättigung ein. Anschließend läßt man die so erhaltene klare Lösung eine Stunde lang stehen und erwärmt dann während 3 Stunden auf 40 °C. Die Lösung wird unter vermindertem Druck eingeengt und der verbleibende feste Rückstand aus Äther kristallisiert, abgesaugt und mit Äther gewaschen.
Ausbeute: 1,1 g (83,1 % der Theorie),
Schmelzpunkt: 227-230 °C
Analog werden folgende Verbindungen erhalten:

(1) 4-Amidino-4'-[4-(cycloheptyloxycarbonyl)-butyrylamino]-biphenyl-hydrochlorid
(2) 4-Amidino-4'-[4-(2-cyclohexyl-äthoxycarbonyl)-butyryl-N-methyl-amino]-biphenyl-hydrochlorid
(3) 4-Amidino-4'-[N-(3-(cyclopentylmethyloxycarbonyl)-propyl)-aminocarbonyl]-biphenyl-hydrochlorid
(4) 4-Amidino-4'-[N-(3-(cyclopentylmethyloxycarbonyl)-propyl)-N-benzyl-aminocarbonyl]-biphenyl-hydrochlorid
(5) 4-Amidino-4'-[4-(2-cyclohexyloxycarbonyl-ethyl)-piperazinocarbonyl]-biphenyl-dihydrochlorid
(6) 4-Amidino-4'-[4-(cyclooctyloxycarbonylmethyl)-piperidinocarbonyl]-biphenyl-hydrochlorid
(7) 4-Amidino-4'-[4-(cyclopentyloxycarbonylmethyl)-piperidinocarbonyl]-biphenyl-hydrochlorid
Schmelzpunkt: >240 °C, sintern ab 219 °C
(8) 4-Amidino-3'-[4-trans-(cyclohexyloxycarbonyl)-cyclohexylaminocarbonyl]-4'-methoxy-biphenyl-hydrochlorid
Schmelzpunkt: 238-241 °C (Zers.)
(9) 4-Amidino-3'-[4-trans-(cyclohexylmethoxycarbonyl)-cyclohexylaminocarbonyl]-4'-methoxy-biphenyl-hydrochlorid
Schmelzpunkt: 247-250 °C (Zers.)
(10) 4-Amidino-4'-[4-(cycloheptyloxycarbonylmethyl)-piperidinocarbonyl]-biphenyl-hydrochlorid
Schmelzpunkt: > 240 °C, sintern ab 215 °C
(11) 4-Amidino-4'-[4-(cyclohexyloxycarbonylmethyl)-piperidinomethyl]-biphenyl-hydrochlorid
(12) 4-Amidino-4'-[4-[(2,2-dimethyl-propyloxy)carbonylmethyl]-piperidinocarbonyl]-biphenyl-hydrochlorid
Schmelzpunkt: 235-240 °C
$R_f$-Wert: 0,73 (Kieselgel, Methylenchlorid/Methanol = 4:1)
(13) 4-Amidino-4'-[4-[(3-pentyloxy)carbonylmethyl]-piperidinocarbonyl]-biphenyl-hydrochlorid
Schmelzpunkt: 205-207 °C
$R_f$-Wert: 0,52 (Kieselgel, Methylenchlorid/Methanol = 4:1)
(14) 4-Amidino-4'-[4-[((R)-2-butyloxy)carbonylmethyl]-piperidinocarbonyl]-biphenyl-hydrochlorid
Schmelzpunkt: 203-205 °C
$R_f$-Wert: 0,56 (Kieselgel, Methylenchlorid/Methanol = 4:1)
(15) 4-Amidino-4'-[4-[((S)-2-butyloxy)carbonylmethyl]-piperidinocarbonyl]-biphenyl-hydrochlorid
Schmelzpunkt: 205-208 °C
$R_f$-Wert: 0,56 (Kieselgel, Methylenchlorid/Methanol = 4:1)
(16) 4-Amidino-4'-[4-[(2-methyl-propyloxy)carbonylmethyl]-piperidinocarbonyl]-biphenyl-hydrochlorid
Man arbeitet ohne Zusatz von Methylenchlorid und läßt 18 Stunden bei Raumtemperatur stehen.
Schmelzpunkt: 218-223 °C
$R_f$-Wert: 0,64 (Kieselgel, Methylenchlorid/Methanol = 4:1)
(17) 4-Amidino-4'-[4-trans-(isopropyloxycarbonyl)-cyclohexylaminocarbonyl]-biphenyl-hydrochlorid
Man arbeitet ohne Zusatz von Methylenchlorid und läßt nach dem Einleiten von Chlorwasserstoff 5 Tage bei Raumtemperatur stehen.
$R_f$-Wert: 0,41 (Kieselgel, Methylenchlorid/Methanol = 8:2)
(18) 4-Amidino-4'-[4-(isopropyloxy)carbonylmethyl]-piperidinocarbonyl]-biphenyl-hydrochlorid
Man verfährt wie unter (16).
Schmelzpunkt: 248-250 °C
$R_f$-Wert: 0,25 (Reversed Phase Platte RP18, Methanol/5%ige Natriumchloridlösung = 6:4)

Beispiel 2

4-Amidino-4'-[4-(cyclohexylmethyloxycarbonylmethyl)-piperidinocarbonyl]-biphenyl

Eine Suspension von 4-Amidino-4'-[4-carboxymethyl-piperidinocarbonyl]-biphenyl, Hydroxymethylcyclohexan und Trimethylchlorsilan im Molverhältnis 1:4:10 wird in trockenem Tetrahydrofuran 36 Stunden lang auf Rückflußtemperatur erhitzt. Hiernach dampft man unter vermindertem Druck zur Trockne ein, verdünnt mit Dichlormethan und wäscht mit 2N Ammoniak und anschließend mit Wasser. Nach Trocknen über Natriumsulfat dampft man unter vermindertem Druck zur Trockne ein. Der Rückstand wird mittels Chromatographie über eine Kieselgelsäule gereinigt.

Analog werden folgende Verbindungen erhalten:

(1) 4-Amidino-4'-[4-(N-methyl-piperidin-4-yloxycarbonylmethyl)-piperidinocarbonyl]-biphenyl
(2) 4-[4-(N-Acetyl-piperidin-4-yloxycarbonylmethyl)-piperidinocarbonyl]-4'-amidino-biphenyl
(3) 4-Amidino-4'-[4-(tetrahydro-4H-pyran-4-yloxycarbonylmethyl)-piperidinocarbonyl]-biphenyl
(4) 4-Amidino-4'-[4-(cyclopentyloxycarbonylmethyl)-piperidinocarbonyl]-biphenyl
(5) 4-Amidino-4'-[5-(N-benzyl-piperidin-4-yloxycarbonyl)-pentyloxy]-biphenyl
(6) 4-Amidino-4'-[4-(tetrahydrofuran-3-yloxycarbonyl)-butyloxyl-biphenyl
(7) 4-Amidino-4'-[4-trans-(N-methyl-piperidin-4-yloxycarbonyl)-cyclohexylaminocarbonyl]-biphenyl
(8) 4-Amidino-3'-[4-trans-(N-methyl-piperidin-4-yloxycarbonyl)-cyclohexylaminocarbonyl]-4'-methoxy-biphenyl

Beispiel 3

4-Amidino-4'-[4-(N-methyl-piperidin-3-yloxycarbonyl)-piperidinocarbonylmethyloxy]-biphenyl-dihydrochlorid

a) 4-[N-Benzyloxycarbonyl-amidino]-4'-[4-(N-methyl-piperidin-3-yloxycarbonyl)-piperidinocarbonylmethyloxy]-biphenyl

Eine Lösung von 4-[N-Benzyloxycarbonyl-amidino]-4'-[4-(carboxymethyl)-piperidinocarbonylmethyloxy]-biphenyl und N-Methyl-piperidin-3-ol im Molverhältnis 1:2 in Dichlormethan/Dimethylformamid wird in Gegenwart einer katalytischen Menge 4-Dimethylamino-pyridin unter Rühren mit einer Lösung von 1,1 Mol-Äquivalenten Dicyclohexyl-carbodiimid in Dichlormethan versetzt. Nach Stehen über Nacht bei Raumtemperatur wird der ausgeschiedene Dicyclohexylharnstoff abfiltriert und die Lösung unter reduziertem Druck zur Trockne eingeengt. Der Rückstand wird in Essigsäure-äthylester aufgenommen und mit 5%iger Natriumbicarbonat-Lösung und anschließend mit Wasser gewaschen und über Natriumsulfat getrocknet. Die verbleibende Lösung wird unter reduziertem Druck eingeengt und der Rückstand mittels Chromatographie über eine Kieselgelsäule gereinigt.

Analog werden folgende Verbindungen erhalten:

(1) 4-[N-Benzyloxycarbonyl-amidino]-4'-[4-(cycloheptyloxycarbonyl)-piperidinocarbonylmethyl]-biphenyl
(2) 4-[N-Benzyloxycarbonyl-amidino]-4'-[4-(dimethylaminocarbonylmethyloxycarbonylmethyl)-piperidinocarbonyl]-biphenyl
(3) 4-[N-Benzyloxycarbonyl-amidino]-4'-[4-(cyclohexylaminocarbonylmethyloxycarbonylmethyl)-piperidinocarbonyl]-biphenyl
(4) 4-[4-(Aminocarbonylmethyloxycarbonylmethyl)-piperidinocarbonyl]-4'-[N-benzyloxycarbonyl-amidino]-biphenyl
(5) 4-[N-Benzyloxycarbonyl-amidino]-3'-[4-trans-(dimethylaminocarbonylmethyloxycarbonyl)-cyclohexylaminocarbonyl]-4'-methoxy-biphenyl
(6) 4-[N-Benzyloxycarbonyl-amidino]-4'-methoxy-3'-[4-trans-(piperidinocarbonylmethyloxycarbonyl)-cyclohexylaminocarbonyl]-biphenyl
(7) 4-[N-Benzyloxycarbonyl-amidino]-4'-methoxy-3'-[4-trans-(morpholinocarbonylmethyloxycarbonyl)-cyclohexylaminocarbonyl]-biphenyl

b) 4-Amidino-4'-[4-(N-methyl-piperidin-3-yloxycarbonyl)-piperidinocarbonylmethyloxy]-biphenyl-dihydrochlorid

Das unter a) gewonnene Produkt wird in einer Lösung, bestehend aus Dimethylformamid, Wasser und 1N Salzsäure, im Volumenverhältnis 20:1:1 in Gegenwart von Palladium auf Kohle (10%ig) bei einem Druck von 5 bar während einer Stunde bei Raumtemperatur hydriert. Nach Absaugen des Katalysators wird unter

vermindertem Druck eingeengt, der Rückstand in Aceton aufgeschlämmt und abgesaugt.

Analog werden folgende Verbindungen erhalten:

(1) 4-Amidino-4'-[4-(cycloheptyloxycarbonyl)-piperidinocarbonylmethyl]-biphenyl-hydrochlorid

(2) 4-Amidino-4'-[4-(dimethylaminocarbonylmethyloxycarbonylmethyl)-piperidinocarbonyl]-biphenyl

(3) 4-Amidino-4'-[4-(cyclohexylaminocarbonylmethyloxycarbonylmethyl)-piperidinocarbonyl]-biphenyl-hydrochlorid

(4) 4-Amidino-4'-[4-(aminocarbonylmethyloxycarbonylmethyl)-piperidinocarbonyl]-biphenyl-hydrochlorid

(5) 4-Amidino-3'-[4-trans-(dimethylaminocarbonylmethyloxycarbonyl)-cyclohexylaminocarbonyl]-4'-methoxy-biphenyl-hydrochlorid

(6) 4-Amidino-4'-methoxy-3'-[4-trans-(piperidinocarbonylmethyloxycarbonyl)-cyclohexylaminocarbonyl]-biphenyl-hydrochlorid

(7) 4-Amidino-4'-methoxy-3'-[4-trans-(morpholinocarbonylmethyloxycarbonyl)-cyclohexylaminocarbonyl]-biphenyl-hydrochlorid

## Beispiel 4

### 4-Amidino-4'-[4-((exo)-norbornyloxycarbonylmethyl)-piperidinocarbonyl]-biphenyl-hydrochlorid

Eine Suspension von 0,4 g (0,0011 Mol) 4-Amidino-4'-[4-carboxymethyl-piperidinocarbonyl]-biphenyl in 10 ml Thionylchlorid wird 2 Stunden lang auf Rückflußtemperatur erhitzt. Die so erhaltene Lösung wird im Vakuum bis zur Trockene eingeengt und der verbleibende Rückstand mit 30 ml Dichlormethan und 0,3 g (exo)-Norborneol versetzt. Nach 12-stündigem Erwärmen auf 40 °C wird im Vakuum eingeengt und der verbleibende Rückstand über eine Kieselgelsäule gereinigt, wobei Dichlormethan/Methanol-Mischungen als Elutionsmittel verwendet werden. Ausbeute: 150 mg (26,6 % der Theorie),
Schmelzpunkt: 175-180 °C

Analog wird folgende Verbindung erhalten:

(1) 4-Amidino-4'-[4-((-)-menthyloxycarbonylmethyl)-piperidinocarbonyl]-biphenyl-hydrochlorid Schmelzpunkt: >260 °C

## Beispiel 5

### 4-Amidino-4'-[4-(pivaloyloxymethyloxycarbonylmethyl)-piperidinocarbonyl]-biphenyl-hydrochlorid

a) 4-(N-Benzyloxycarbonyl-amidino)-4'-[4-(pivaloyloxymethyloxycarbonylmethyl)-piperidinocarbonyl]-biphenyl

Eine Mischung von 1,7 g (0,0034 Mol) 4-[N-Benzyloxycarbonylamidino]-4'-[4-(carboxymethyl)-piperidinocarbonyl]-biphenyl, 1 ml (0,0068 Mol) Pivalinsäure-chlormethylester, 1,1 g (0,0068 Mol) Kaliumjodid, 0,7 g (0,0068 Mol) Kaliumbicarbonat und 1 g (0,0068 Mol) Kaliumcarbonat in 40 ml Dimethylformamid wird während 2 Tagen bei Raumtemperatur gerührt. Anschließend wird in 150 ml Wasser gegossen und die so entstandene Suspension 3 mal mit Essigsäure-äthylester extrahiert. Die vereinigten organischen Phasen werden über Natriumsulfat getrocknet und unter reduziertem Druck zur Trockne eingeengt. Der verbleibende Rückstand wird mittels Chromatographie über eine Kieselgelsäule gereinigt, wobei Cyclohexan-Essigester-Mischungen als Solventien dienen.

Man erhält so 1,45 g (68,5 % der Theorie) als schaumiges Produkt, das ohne weitere Reinigung für die nächste Synthesestufe verwendet wird.

$R_f$-Wert: 0,5 (Kieselgel; Methylenchlorid/Äthanol = 9/1)

Analog werden folgende Verbindungen erhalten:

(1) 4-[4-[(1-(Äthoxycarbonyloxy)-äthoxycarbonyl)-methyl]-piperidinocarbonyl]-4'-[N-benzyloxycarbonyl-amidino]-biphenyl Öl, $R_f$-Wert 0,65 (Kieselgel; Methylenchlorid/Äthanol = 9:1)

(2) 4-[N-Benzyloxycarbonyl-amidino]-4'-[4-(pivaloyloxymethyloxycarbonylmethyliden)-piperidinocarbonyl]-biphenyl

(3) 4-[4-[(1-(Äthoxycarbonyloxy)-äthoxycarbonyl)-methyl]-piperidinomethyl]-4'-[N-benzyloxycarbonyl-amidino]-biphenyl

(4) 4-[N-Benzyloxycarbonyl-amidino]-4'-[(2-(pivaloyloxymethyloxycarbonyl)-ethyl)-aminocarbonylmethyl]-biphenyl

(5) 4-[2-(1-Äthoxycarbonyloxy)-äthoxycarbonyl)-aminocarbonylmethyloxy]-4'-[N-benzyloxycarbonyl-amidino]-biphenyl

(6) 4-[N-Benzyloxycarbonyl-amidino]-4'-[1-[2-(4-methoxyphenyl)-ethylaminocarbonyl]-2-(pivaloyloxymethyloxycarbonyl)-ethylamino]-carbonylmethyl]-biphenyl

(7) 4-[Benzyloxycarbonyl-amidino]-4'-[4-[1-(cyclohexyloxycarbonyloxy)-ethyloxycarbonylmethyl]-piperidinocarbonyl]-biphenyl

(8) 4-[Benzyloxycarbonyl-amidino]-4'-methoxy-3'-[4-trans-(pivaloyloxymethyloxycarbonyl)-cyclohexylaminocarbonyl]-biphenyl

(9) 4-[N-Allyloxycarbonyl-amidino]-4'-[4-(pivaloyloxymethyloxycarbonylmethyliden)-piperidinocarbonyl]-biphenyl

(10) 3-[4-trans-[1-(Äthoxycarbonyloxy)-äthoxycarbonyl]-cyclohexylaminocarbonyl]-4'-[N-benzyloxycarbonyl-amidino]-4-methoxy-biphenyl

b) 4-Amidino-4'-[4-(pivaloyloxymethyloxycarbonylmethyl)-piperidinocarbonyl]-biphenyl-hydrochlorid

Das unter a) gewonnene Produkt (1,43 g = 0,0023 Mol) wird in einer Mischung aus 20 ml Dimethylformamid und 2,3 ml 0,5N Salzsäure gelöst. Diese Lösung wird in Gegenwart von 0,2 g Palladium auf Kohle (10%ig) bei einem Druck von 5 bar während 2,5 Stunden bei Raumtemperatur hydriert. Nach Absaugen des Katalysators wird ein pH-Wert von 3 eingestellt und die Lösung unter vermindertem Druck zur Trockne eingeengt. Der Rückstand wird in Aceton aufgeschlämmt. Das Festprodukt wird abgesaugt.
Ausbeute: 0,8 g (69,8 % der Theorie),
Schmelzpunkt: 168-172 °C
Analog werden folgende Verbindungen erhalten:

(1) 4-[4-[(1-(Äthoxycarbonyloxy)-äthoxycarbonyl)-methyl]-piperidinocarbonyl]-4'-amidino-biphenyl-hydrochlorid mit 1-Chloräthyl-äthyl-carbonat
Schmelzpunkt: 178-182 °C

(2) 4-Amidino-[4'-[4-(pivaloyloxymethyloxycarbonylmethyliden)-piperidinocarbonyl]-biphenyl-hydrochlorid mit Pivalinsäure-chlormethylester

(3) 4-[4-[(1-(Äthoxycarbonyloxy)-äthoxycarbonyl)-methyl]-piperidinomethyl]-4'-amidino-biphenyl-hydrochlorid

(4) 4-Amidino-4'-[(2-(pivaloyloxymethyloxycarbonyl)-ethyl)-aminocarbonylmethyl]-biphenyl

(5) 4'-Amidino-4-[2-(1-Äthoxycarbonyloxy)-äthoxycarbonyl)-aminocarbonylmethyloxy]-biphenyl-hydrochlorid

(6) 4-Amidino-4'-[1-[2-(4-methoxyphenyl)-ethylaminocarbonyl]-2-(pivaloyloxymethyloxycarbonyl)-ethylamino]-carbonylmethyl]-biphenyl

(7) 4-Amidino-4'-[4-[1-(cyclohexyloxycarbonyloxy)-ethyloxycarbonylmethyl]-piperidinocarbonyl]-biphenyl mit 1-Chloräthyl-cyclohexyl-carbonat

(8) 4-Amidino-4'-methoxy-3'-[4-trans-(pivaloyloxymethyloxycarbonyl)-cyclohexylaminocarbonyl]-biphenyl

(9) 4'-Amidino-3-[4-trans-[1-(äthoxycarbonyloxy)-äthoxycarbonyl]-cyclohexylaminocarbonyl]-4-methoxy-biphenyl

## Beispiel 6

### 4-Amidino-4'-[4-(phthalid-3-yloxycarbonylmethyl)-piperidinocarbonyl]-biphenyl

a) [4-(N-Allyloxycarbonyl-amidino]-4'-[4-(methoxycarbonylmethyl)-piperidinocarbonyl]-biphenyl

Zu einer Lösung von 2,5 g (0,006 Mol) 4-Amidino-4'-[4-(methoxycarbonylmethyl)-piperidinocarbonyl)-biphenyl in 250 ml Dichlormethan gibt man bei Raumtemperatur und unter Rühren 0,75 ml (0,007 Mol) Chlorameisensäure-allyester und 13 ml (0,013 Mol) 1N Natronlauge. Diese Mischung wird während 3 Stunden bei Raumtemperatur gerührt, anschließend läßt man über Nacht bei Raumtemperatur stehen. Die organische Phase wird abgetrennt, über Natriumsulfat getrocknet und mit 2 g Aktivkohle verrührt. Nach Abfiltrieren des Trockenmittels und der Aktivkohle wird die Lösung unter reduziertem Druck zur Trockne eingeengt. Der Rückstand wird in Dichlormethan/Methanol = 1:1 gelöst; man gibt 20 ml Isopropanol zu und engt auf 20 ml ein. Nach Zusatz von Äther tritt Kristallisation ein. Das Kristallisat wird abgesaugt und mit Äther gewaschen. Ausbeute: 2,4 g (86,3 % der Theorie),
Schmelzpunkt: 148-152 °C
Analog wird folgende Verbindung erhalten:

(1) 4-[N-Allyloxycarbonyl-amidino]-4'-[[1-[2-(4-methoxy-phenyl)-ethylaminocarbonyl]-2-methoxycarbonyl-ethyl]-aminocarbonyl]-biphenyl

b) 4-[N-Allyloxycarbonyl-amidino]-4'-[4-(carboxymethyl)-piperidinocarbonyl]-biphenyl

1,95 g (0,042 Mol) der unter a) hergestellten Verbindung werden in 20 ml Tetrahydrofuran und 4 ml Wasser gelöst. Zu dieser Lösung gibt man 21 ml einer 1 molaren Lithiumhydroxid-Lösung (0,021 Mol) und läßt während 2 Stunden bei Raumtemperatur stehen. Anschließend gießt man in eine wässrige Kaliumhydrogensulfat-Lösung, stellt den pH-Wert auf 6.5 ein und extrahiert erschöpfend mit Essigester. Die vereinigten Essigester-Phasen werden getrocknet und eingeengt. Der Rückstand wird mit Äther verrieben und abgesaugt.
Ausbeute: 1,2 g (63,6 % der Theorie),
Schmelzpunkt: 189-192 °C (Zers.)
Analog wird folgende Verbindung erhalten:

(1) 4-[N-Allyloxycarbonyl-amidino]-4'-[[1-[2-(4-methoxy-phenyl)-ethylaminocarbonyl]-2-hydroxycarbonyl-ethyl]-aminocarbonyl]-biphenyl

c) 4-[N-Allyloxycarbonyl-amidino]-4'-[4-(phthalid-3-yloxycarbonylmethyl)-piperidinocarbonyl]-biphenyl

0,3 g (0,0007 Mol) der unter b) hergestellten Verbindung werden in 30 ml Dimethylformamid gelöst. Diese Lösung wird mit 0,4 g (0,004 Mol) Kaliumhydrogencarbonat versetzt und hierzu gibt man unter Rühren und bei 0-5 °C, 0,71 g (0,0033 Mol) 3-Bromphthalid und setzt das Rühren während 3 Stunden bei dieser Temperatur fort. Nach dieser Zeit gießt man in Eiswasser und extrahiert mit Essigester. Die vereinigten Essigester-Phasen werden mit Wasser gewaschen, über Natriumsulfat getrocknet und mit reduziertem Druck eingeengt. Der Rückstand wird mittels Chromatographie über Kieselgel gereinigt.

Analog wird folgende Verbindung erhalten:

(1) 4-[N-Allyloxycarbonyl-amidino]-4'-[[1-[2-(4-methoxy-phenyl)-ethylaminocarbonyl]-2-(phthalid-3-yloxcarbonyl)-ethyl]-aminocarbonyl]-biphenyl

d) 4-Amidino-4'-[4-(phthalid-3-yloxycarbonylmethyl)-piperidinocarbonyl]-biphenyl

Die unter c) erhaltene Verbindung wird in Tetrahydrofuran gelöst. Unter Intertgas-Atmosphäre tropft man unter Rühren 1 Äquivalent Morpholin und 0,1 Äquivalent Tetrakis-(triphenylphosphin)-palladium(O) zu und setzt das Rühren während einer weiteren Stunde fort.

Analog wird folgende Verbindung erhalten:

(1) 4-Amidino-4'-[[1-[2-(4-methoxyphenyl)-ethylaminocarbonyl]-2-(phthalid-3-yloxycarbonyl)-ethyl]-aminocarbonyl]-biphenyl

Beispiel 7

4-[4-(Cyclohexyloxycarbonylmethyl)-piperidinocarbonyl]-4'-[N-methoxycarbonyl-amidino]-biphenyl

0,6 g (0,0012 Mol) 4-Amidino-4'-[4-(cyclohexyloxycarbonylmethyl)-piperidinocarbonyl]-biphenyl-hydrochlorid werden in 200 ml Dichlormethan suspendiert. Unter Rühren versetzt man mit 0,1 g (0,0012 Mol) Chlorameisensäure-methylester und 24,8 ml (0,0025 Mol) 0,1N Natronlauge und rührt anschließend weitere 2 Stunden, wobei eine Lösung entsteht. Die Wasser-Phase wird abgetrennt, die Dichlormethanlösung über Natriumsulfat getrocknet und zur Trockne eingeengt. Der Rückstand wird mit Äther verrieben, abgesaugt und mit Äther gewaschen. Ausbeute: 0,44 g (76,2 % der Theorie),
Schmelzpunkt: 218-220 °C
Analog werden folgende Verbindungen erhalten:

(1) 4-[N-Allyloxy-carbonyl-amidino]-4'-[4-(cyclohexyloxycarbonylmethyl)-piperidinocarbonyl]-biphenyl
(2) 4-[N-Methoxycarbonyl-amidino]-4'-[4-(N-methyl-piperidin-4-yloxycarbonylmethyl)-piperidinocarbonyl]-biphenyl
(3) 4-[N-Methoxycarbonyl-amidino]-4'-[4-(tetrahydro-4H-pyran-4-yloxycarbonylmethyl)-piperidinocarbonyl]-biphenyl
(4) 4-[N-(Cyclohexylmethyloxycarbonylmethyl)-piperidinocarbonyl]-biphenyl
(5) 4-[4-(Cyclopentyloxycarbonylmethyliden)-piperidinocarbonyl]-4'-[N-methoxycarbonyl-amidino]-biphenyl
(6) 4'-[4-(4-Cycloheptyloxycarbonyl-butyrylamino)]-4-[N-methoxycarbonyl-amidino]-biphenyl
(7) 4-[4-(Cyclohexyloxycarbonylmethyl)-piperazino)-carbonyl]-4'-[N-methoxycarbonyl-amidino]-biphenyl

(8) 4-[N-Methoxycarbonyl-amidino]-4'-[4-trans-(N-methylpiperidin-4-yloxycarbonyl)-cyclohexylaminocarbonyl]-biphenyl

(9) 3-[4-trans-Cyclohexyloxycarbonyl-cyclohexylaminocarbonyl]-4-methoxy-4'-[N-methoxcarbonyl-amidino]-biphenyl

(10) 4-[N-Methoxycarbonyl-amidino]-4'-methoxy-3'-[4-trans-(N-methyl-piperidin-4-yloxycarbonyl)-cyclohexylaminocarbonyl]-biphenyl

(11) 4-[4-(Methoxycarbonylmethyl)-piperidinocarbonyl]-4'-[N-(pivaloyloxymethoxycarbonyl)-amidino]-biphenyl

Es wird Kohlensäure-(pivaloyloxy-methyl)-(4-nitrophenyl)-ester und N-Ethyl-diisopropylamin verwendet.

(12) 4-(N-(Acetyloxymethoxycarbonyl)-amidino]-4'-[4-(methoxycarbonylmethyl)-piperidinocarbonyl]-biphenyl

Es wird Kohlensäure-acetyloxymethyl-(4-nitrophenyl)-ester und N-Ethyl-diisopropylamin verwendet.

(13) 4-[N-(1-Acetyloxy-ethoxycarbonyl)-amidino]-4'-[4-(methoxycarbonylmethyl)-piperidinocarbonyl]-biphenyl

Es wird Kohlensäure-acetyloxyethyl-(4-nitrophenyl)-ester und N-Ethyl-diisopropylamin verwendet.

(14) 4-[4-[(2,2-Dimethyl-propyloxy)carbonylmethyl]-piperidinocarbonyl]-4'-(N-methoxycarbonyl-amidino)-biphenyl

Schmelzpunkt: 168-170 °C

$R_f$-Wert: 0,50 (Kieselgel, Essigester)

(15) 4-(N-Methoxycarbonyl-amidino)-4'-[4-[(3-pentyloxy)carbonylmethyl]-piperidinocarbonyl]-biphenyl

Schmelzpunkt: 148-152 °C

$R_f$-Wert: 0,58 (Kieselgel, Methylenchlorid/Methanol = 9:1)

(16) 4-[4-[((R)-2-Butyloxy)carbonylmethyl]-piperidinocarbonyl]-4'-(N-methoxycarbonyl-amidino)-biphenyl

Schmelzpunkt: 135-138 °C

$R_f$-Wert: 0,56 (Kieselgel, Methylenchlorid/Methanol = 9:1)

(17) 4-[4-[((S)-2-Butyloxy)carbonylmethyl]-piperidinocarbonyl]-4'-(N-methoxycarbonyl-amidino)-biphenyl

Schmelzpunkt: 130-132 °C

$R_f$-Wert: 0,51 (Kieselgel, Methylenchlorid/Methanol = 9:1)

(18) 4-(N-Methoxycarbonyl-amidino)-4'-[4-[(2-methyl-propyloxy)carbonylmethyl]-piperidinocarbonyl]-biphenyl

Schmelzpunkt: 146-152 °C

$R_f$-Wert: 0,51 (Kieselgel, Methylenchlorid/Methanol = 9:1)

(19) 4-[4-(Isopropyloxy)carbonylmethyl]-piperidinocarbonyl]-4'-(N-methoxycarbonyl-amidino)-biphenyl

Schmelzpunkt: 146-148 °C

$R_f$-Wert: 0,38 (Kieselgel, Methylenchlorid/Methanol = 9:1)

## Beispiel 8

### 4-Amidino-4'-[4-(piperidin-4-yloxycarbonylmethyl)-piperidinocarbonyl]-biphenyl-hydrochlorid

a) 4-Amidino-4'-[4-(N-benzyloxycarbonyl-piperidin-4-yloxycarbonylmethyl)-piperidinocarbonyl]-biphenyl

Eine Suspension von 4-Amidino-4'-[4-carboxymethyl-piperidinocarbonyl]-biphenyl, N-Benzyloxycarbonyl-piperidin-4-ol und Trimethylchlorsilan im Molverhältnis 1:4:10 wird in absolutem Tetrahydrofuran 36 Stunden lang auf Rückflußtemperatur erhitzt. Anschließend wird im Vakuum zur Trockene eingeengt, in Dichlormethan aufgenommen und die organische Phase mit 2N Ammoniak und anschließend mit Wasser gewaschen. Nach Trocknen der organischen Phase über Natriumsulfat wird im Vakuum eingeengt und der erhaltene Rückstand mittels Chromatographie über Kieselgel gereinigt.

b) 4-Amidino-4'-[4-(piperidin-4-yloxycarbonylmethyl)-piperidinocarbonyl]-biphenyl-hydrochlorid

Das unter a) hergestellte Produkt wird in Dimethylformamid/0,5N Salzsäure (10:1) gelöst und anschließend in Gegenwart von 10%igem Palladium auf Kohle bei einem Wasserstoffdruck von 5 bar bei Raumtemperatur hydriert.

Analog werden folgende Verbindungen erhalten:

(1) 4-Amidino-4'-[4-trans-(N-benzyloxycarbonyl-piperidin-4-yloxycarbonyl)-cyclohexylaminocarbonyl]-biphenyl

(2) 4-Amidino-4'-[4-trans-(N-benzyloxycarbonyl-piperidin-4-yloxycarbonyl)-cyclohexyl-N-methyl-aminocarbonyl]-biphenyl
(3) 4-Amidino-4'-[4-trans-(piperidin-4-yloxycarbonyl)-cyclohexylaminocarbonyl]-biphenyl-dihydrochlorid
(4) 4-Amidino-4'-[4-trans-(piperidin-4-yloxycarbonyl)-cyclohexyl-N-methyl-aminocarbonyl]-biphenyl-dihydrochlorid

Beispiel 9

4-[N-Methoxycarbonyl-amidino]-4'-[4-(piperidin-4-yloxycarbonylmethyl)-piperidinocarbonyl]-biphenyl-hydrochlorid

a) 4-[4-(N-Benzyloxycarbonyl-piperidin-4-yloxycarbonylmethyl)-piperidinocarbonyl]-4'-[N-methoxycarbonylamidino]-biphenyl

4-Amidino-4'-[4-(N-benzyloxycarbonyl-piperidin-4-yloxycarbonylmethyl)-piperidinocarbonyl]-biphenyl wird in Dichlormethan suspendiert und unter Rühren mit einer äquimolaren Menge Chlorameisensäuremethylester in Gegenwart einer ebenfalls äquivalenten Menge 0,1N Natronlauge bei Raumtemperatur zur Reaktion gebracht. Die Dichlormethan-Lösung wird mit Wasser gewaschen, getrocknet und eingedampft. Der Rückstand wird mittels Säulenchromatographie über Kieselgel gereinigt.

Analog wird folgende Verbindung erhalten:
(1) 4'-[4-trans-(N-Benzyloxycarbonyl-piperidin-4-yloxycarbonyl)-cyclohexylaminocarbonyl]-4-[N-methoxycarbonyl-amidino]-biphenyl

b) 4-[N-Methoxycarbonyl-amidino]-4-[4-(piperidin-4-yloxycarbonylmethyl)-piperidinocarbonyl]-biphenyl-hydrochlorid

Das unter c) gewonnene Produkt wird wie in Beispiel 8b beschrieben in Gegenwart von Palladium/Kohle (10%ig) hydriert.

Analog wird folgende Verbindung erhalten:
(1) 4-[N-Methoxycarbonyl-amidino]-4'-[trans-(piperidin-4-yloxycarbonyl)-cyclohexylaminocarbonyl-biphenyl

Beispiel 10

Trockenampulle mit 2,5 mg Wirkstoff pro 1 ml

Zusammensetzung:

| | |
|---|---|
| Wirkstoff | 2,5 mg |
| Mannitol | 50,0 mg |
| Wasser für Injektionszwecke ad | 1,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 11

Trockenampulle mit 35 mg Wirkstoff pro 2 ml

Zusammensetzung:

| | |
|---|---|
| Wirkstoff | 35,0 mg |
| Mannitol | 100,0 mg |
| Wasser für Injektionszwecke ad | 2,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 12

Tablette mit 50 mg Wirkstoff

Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 13

Tablette mit 350 mg Wirkstoff

Zusammensetzung:

| | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 14

Kapseln mit 50 mg Wirkstoff

Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

Beispiel 15

Kapseln mit 350 mg Wirkstoff

Zusammensetzung:

| | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

**Patentansprüche**

**1.** Biphenylderivate der allgemeinen Formel

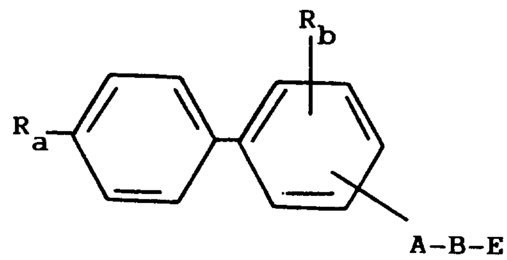

,(I)

in der

mit der Maßgabe, daß $R_a$ keine gegebenenfalls durch eine Alkoxycarbonylgruppe mit insgesamt 2 oder 3 Kohlenstoffatomen oder keine durch eine Benzyloxycarbonylgruppe substituierte Amidinogruppe darstellt, wenn E eine Alkoxycarbonylgruppe mit insgesamt 2 oder 3 Kohlenstoffatomen oder eine Benzyloxycarbonylgruppe darstellt,

$R_a$ eine gegebenenfalls durch eine $R_1$-CO-O-($R_2CR_3$)-O-CO-, Alkoxycarbonyl-, Phenylalkoxycarbonyl-, Alkenyloxycarbonyl- oder Phenylalkenyloxycarbonylgruppe substituierte Amidinogruppe, wobei

die vorstehend erwähnten Alkoxyteile jeweils 1 bis 6 Kohlenstoffatome und der vorstehend erwähnte Alkenylteil 3 bis 5 Kohlenstoffatome enthalten kann,

$R_1$ eine geradkettige oder verzweigte Alkyl- oder Alkoxygruppe mit jeweils 1 bis 5 Kohlenstoffatomen, eine Cycloalkyl- oder Cycloalkoxygruppe mit jeweils 5 bis 7 Kohlenstoffatomen, eine Phenylalkyl-, Phenylalkoxy-, Phenyl- oder Phenoxygruppe,

$R_2$ ein Wasserstoffatom, eine Alkylgruppe mit 1 bis 6 Kohlenstoffatomen, eine Cycloalkylgruppe mit 3 bis 7 Kohlenstoffatomen oder eine Phenylgruppe und

$R_3$ ein Wasserstoffatom oder eine Alkylgruppe mit 1 bis 6 Kohlenstoffatomen darstellen,

$R_b$ ein Wasserstoffatom, eine Alkyl-, Hydroxy- oder Alkoxygruppe,

A eine Bindung, eine $-CH_2-$, -CO-, $-CH_2CO-$ oder $-O-CH_2CO-$Gruppe, wobei die $-CH_2CO-$ und $-O-CH_2CO-$Gruppe jeweils über die Carbonylgruppe mit dem Rest B verknüpft ist, und

B eine $-NR_4-CH_2CH_2-X-(CH_2)_n-$Gruppe, in der X eine Bindung, eine $-HCR_5-$ oder $-NR_5-$Gruppe darstellt, eine $-NR_4-CH_2CH_2-R_6C=CH-$, $-NR_4-CO-(CH_2)_m-$ oder -Y-W-Gruppe, in der Y eine $NR_4$-Gruppe oder auch, wenn A eine Bindung darstellt, ein Sauerstoffatom darstellt, wobei die vorstehenden Y- oder $NR_4$-Reste jeweils mit dem Rest A verknüpft sind sowie

n die Zahl 0 oder 1,

m die Zahl 2, 3, 4 oder 5,

$R_4$ ein Wasserstoffatom, eine Alkyl- oder Phenylalkylgruppe,

$R_5$ ein Wasserstoffatom oder

$R_4$ und $R_5$ sowie $R_4$ und $R_6$ zusammen jeweils eine Ethylengruppe und

W eine geradkettige Alkylengruppe mit 2 bis 5 Kohlenstoffatomen, wobei die Ethylengruppe durch eine Phenylalkylaminocarbonylgruppe substituiert sein kann, oder eine 1,4-Cyclohexylengruppe darstellen,

E eine Alkoxycarbonylgruppe, in der der Alkoxyteil 2 bis 6 Kohlenstoffatome enthalten kann, eine Phenylalkoxycarbonylgruppe, eine Cycloalkoxycarbonylgruppe mit insgesamt 4 bis 10 Kohlenstoffatomen, in welcher der Cycloalkylteil mit 5 bis 8 Kohlenstoffatomen zusätzlich durch 1 oder 2 Alkylgruppen substituiert sein kann, eine Cycloalkoxycarbonylgruppe mit insgesamt 6 bis 8 Kohlenstoffatomen, in der im Cycloalkoxyteil eine Methylengruppe in 3- oder 4-Stellung durch ein Sauerstoffatom oder durch eine gegebenenfalls durch eine Alkyl-, Phenylalkyl- oder Phenylalkoxycarbonylgruppe oder durch eine Alkanoylgruppe mit 2 bis 6 Kohlenstoffatomen substituierte Iminogruppe ersetzt ist und der Cycloalkoxyteil zusätzlich durch 1 oder 2 Alkylgruppen substituiert sein kann, eine Cycloalkenyloxycarbonylgruppe, in der der Cycloalkenylteil 5 bis 8 Kohlenstoffatome enthalten kann, eine Alkenyloxycarbonyl-, Phenylalkenyloxycarbonyl-, Alkinyloxycarbonyl- oder Phenylalkinylozycarbonylgruppe mit der Maßgabe, daß keine Bindung an das Sauerstoffatom von einem Kohlenstoffatom ausgeht, welches eine Doppel- oder Dreifachbindung trägt und in denen der Alkenyl- und Alkinylteil jeweils 3 bis 5 Kohlenstoffatome enthalten kann, eine Cycloalkylalkoxycarbonylgruppe, in der der Cycloalkylteil 3 bis 8 Kohlenstoffatome enthalten kann, eine Bicycloalkoxycarbonylgruppe mit insgesamt 8 bis 10 Kohlenstoffatomen, die im Bicycloalkylteil zusätzlich durch ein oder zwei Alkylgruppen substituiert sein kann, eine $R_1$-CO-O-($R_2CR_3$)-O-CO-Gruppe, in der $R_1$ bis $R_3$ wie vorstehend erwähnt definiert sind, eine $R_7-CO-CH_2-O-CO-$Gruppe, in der $R_7$ eine Aminogruppe, die durch 1 oder 2 Alkylgruppen mit jeweils 1 bis 6 Kohlenstoffatomen oder durch eine Cycloalkylgruppe mit 3 bis 8 Kohlenstoffatomen substituiert sein kann, eine

Pyrrolidinyl-, Piperidinyl-, Hexamethylenimino-, Morpholinyl- oder N-Alkyl-piperazinylgruppe darstellt, oder eine 1,3-Dihydro-3-oxo-1-isobenzfuranyloxycarbonylgruppe,
wobei, soweit nichts anderes erwähnt wurde, die vorstehend erwähnten Alkyl- und Alkoxyteile jeweils 1 bis 3 Kohlenstoffatome enthalten können sowie
die bei der Definition der Reste $R_a$, B und E vorstehend erwähnten Phenylkerne jeweils durch Fluor-, Chlor- oder Bromatome, durch Methyl-, Ethyl-, n-Propyl-, Isopropyl-, Hydroxy-, Methoxy-, Ethoxy-, n-Propoxy-, Isopropoxy-, Trifluormethyl-, Nitro-, Amino-, Methylamino-, Ethylamino-, n-Propylamino-, Isopropylamino-, Acetamino-, Methansulfonylamino-, Carboxy-, Methoxycarbonyl-, Ethoxycarbonyl-, n-Propoxycarbonyl- oder Isopropoxycarbonylgruppe mono- oder disubstituiert sein und die Substituenten gleich oder verschieden sein können, bedeuten,
deren Stereoisomere, einschließlich ihrer Gemische und deren Salze.

**2.** Biphenylderivate der allgemeinen Formel I gemäß Anspruch 1, in denen mit der Maßgabe, daß $R_a$ keine gegebenenfalls durch eine Alkoxycarbonylgruppe mit insgesamt 2 oder 3 Kohlenstoffatomen oder keine durch eine Benzyloxycarbonylgruppe substituierte Amidinogruppe darstellt, wenn E eine Alkoxycarbonylgruppe mit insgesamt 2 oder 3 Kohlenstoffatomen oder eine Benzyloxycarbonylgruppe darstellt,
$R_a$ eine gegebenenfalls durch eine $R_1$-CO-O-($R_2CR_3$)-O-CO-, Alkoxycarbonyl-, Phenylalkoxycarbonyl- oder Alkenyloxycarbonylgruppe substituierte Amidinogruppe, wobei
die vorstehend erwähnten Alkoxylteile jeweils 1 bis 3 Kohlenstoffatome und der vorstehend erwähnte Alkenylteil 3 oder 4 Kohlenstoffatome enthalten kann,
$R_1$ eine geradkettige oder verzweigte Alkylgruppe mit 1 bis 5 Kohlenstoffatomen, eine Phenylalkylgruppe mit 1 bis 3 Kohlenstoffatomen im Alkylteil, eine Alkoxygruppe mit 1 bis 3 Kohlenstoffatomen, eine Cycloalkyl- oder Cycloalkoxygruppe mit jeweils 5 bis 7 Kohlenstoffatomen oder eine Phenylgruppe,
$R_2$ ein Wasserstoffatom, eine Alkylgruppe mit 1 bis 3 Kohlenstoffatomen, eine Cycloalkylgruppe mit 5 bis 7 Kohlenstoffatomen oder eine Phenylgruppe und
$R_3$ ein Wasserstoffatom darstellen,
$R_b$ ein Wasserstoffatom, eine Hydroxy- oder Alkoxygruppe mit 1 bis 3 Kohlenstoffatomen,
A eine Bindung, eine $-CH_2-$, -CO-, $-CH_2CO-$ oder $-O-CH_2CO$-Gruppe, wobei die $-CH_2CO-$ und $-O-CH_2CO$-Gruppe jeweils über die Carbonylgruppe mit dem Rest B verknüpft ist, und
B eine $-NR_4-CH_2CH_2-X-(CH_2)_n$-Gruppe, in der X eine Bindung, eine $-HCR_5-$ oder $-NR_5$-Gruppe darstellt, eine $-NR_4-CH_2CH_2-R_6C=CH-$, $-NR_4-CO-(CH_2)_m-$ oder -Y-W-Gruppe, in der Y eine $-NR_4$-Gruppe oder auch, wenn A eine Bindung darstellt, ein Sauerstoffatom darstellt, wobei die vorstehenden Y- oder $NR_4$-Reste jeweils mit dem Rest A verknüpft sind sowie
m die Zahl 2, 3, 4 oder 5,
n die Zahl 0 oder 1,
$R_4$ ein Wasserstoffatom, eine Alkylgruppe mit 1 bis 3 Kohlenstoffatomen oder eine Benzylgruppe,
$R_5$ ein Wasserstoffatom oder
$R_4$ und $R_5$ sowie $R_4$ und $R_6$ zusammen jeweils eine Ethylengruppe und
W eine geradkettige Alkylengruppe mit 2 bis 5 Kohlenstoffatomen, wobei die Ethylengruppe in $\alpha$-Stellung durch eine Phenylethylaminocarbonylgruppe substituiert sein kann, oder eine 1,4-Cyclohexylengruppe darstellen,
E eine Alkoxycarbonylgruppe, in der der Alkoxyteil 2 bis 6 Kohlenstoffatome enthalten kann, eine Benzyloxycarbonylgruppe, eine Cycloalkoxycarbonylgruppe mit insgesamt 4 bis 10 Kohlenstoffatomen, in welcher der Cycloalkylteil mit 5 bis 8 Kohlenstoffatomen zusätzlich durch 1 oder 2 Alkylgruppen mit jeweils 1 bis 3 Kohlenstoffatomen substituiert sein kann, eine Cycloalkoxycarbonylgruppe mit insgesamt 6 bis 8 Kohlenstoffatomen, in der im Cycloalkoxyteil eine Methylengruppe in 3- oder 4-Stellung durch ein Sauerstoffatom oder durch eine gegebenenfalls durch eine Methyl-, Benzyl- oder Benzyloxycarbonylgruppe oder durch eine Alkanoylgruppe mit 2 bis 4 Kohlenstoffatomen substituierte Iminogruppe ersetzt ist, eine Cycloalkylalkoxycarbonylgruppe, in der der Cycloalkylteil 5 bis 7 Kohlenstoffatome und der Alkoxyteil 1 oder 2 Kohlenstoffatome enthalten kann, eine Bicycloalkoxycarbonylgruppe mit insgesamt 8 oder 9 Kohlenstoffatomen, die im Bicycloalkylteil zusätzlich durch ein oder zwei Methylgruppen substituiert sein kann, eine $R_1$-CO-O-($R_2CR_3$)-O-CO-Gruppe, in der $R_1$ bis $R_3$ wie vorstehend erwähnt definiert sind, eine $R_7-CO-CH_2-O-CO$-Gruppe, in der $R_7$ eine Aminogruppe, die durch 1 oder 2 Alkylgruppen mit jeweils 1 bis 3 Kohlenstoffatomen oder durch eine Cycloalkylgruppe mit 5 bis 7 Kohlenstoffatomen substituiert sein kann, eine Pyrrolidinyl-, Piperidinyl-, Hexamethylenimino-, Morpholinyl- oder N-Methyl-piperazinylgruppe darstellt, oder eine 1,3-Dihydro-3-oxo-1-isobenzfuranyloxycarbonylgruppe bedeuten,
wobei die bei der Definition der Reste $R_a$, B und E vorstehend erwähnten Phenylkerne jeweils durch Fluor-, Chlor- oder Bromatome, durch Methyl-, Hydroxy-, Methoxy-, Trifluormethyl-, Nitro-, Amino-, Methylamino-, Acetamino-, Methansulfonylamino-, Carboxy-, Methoxycarbonyl-, Ethoxycarbonyl-, n-Propoxycarbonyl- oder Isopropoxycarbonylgruppe mono- oder disubstituiert sein und die Substituenten gleich oder verschieden sein können,
deren Stereoisomere, einschließlich ihrer Gemische und deren Salze.

**3.** Biphenylderivate der allgemeinen Formel I gemaß Anspruch 1, in denen mit der Maßgabe, daß $R_a$ keine gegebenenfalls durch eine Alkoxycarbonylgruppe mit insgesamt 2 oder 3 Kohlenstoffatomen oder keine durch eine Benzyloxycarbonylgruppe substituierte Amidinogruppe darstellt, wenn E Alkoxycarbonylgruppe mit insgesamt 2 oder 3 Kohlenstoffatomen oder eine Benzyloxycarbonylgruppe darstellt,
$R_a$ eine gegebenenfalls durch eine $R_1$-CO-O-($R_2CR_3$)-O-CO-, Alkoxycarbonyl-, Benzyloxycarbonyl- oder Allyloxycarbonylgruppe substituierte Amidinogruppe, wobei
der vorstehend erwähnte Alkoxyteil 1 bis 3 Kohlenstoffatome enthalten kann,
$R_1$ eine geradkettige oder verzweigte Alkylgruppe mit 1 bis 4 Kohlenstoffatomen, eine Alkoxygruppe mit 1 bis 3 Kohlenstoffatomen oder eine Cyclohexyloxygruppe,
$R_2$ ein Wasserstoffatom oder eine Methylgruppe und
$R_3$ ein Wasserstoffatom darstellen,
$R_b$ ein Wasserstoffatom oder eine Methoxygruppe,
A eine Bindung, eine $-CH_2-$, -CO-, $-CH_2CO-$ oder $-O-CH_2CO-$Gruppe, wobei die $-CH_2CO-$ und $-O-CH_2CO-$Gruppe jeweils über die Carbonylgruppe mit dem Rest B verknüpft ist, und
B eine $-NR_4-CH_2CH_2-X-(CH_2)_n-$Gruppe, in der X eine Bindung, eine $-HCR_5-$ oder $-NR_5-$Gruppe darstellt, eine $-NR_4-CH_2CH_2-R_6C=CH-$, $-NR_4-CO-(CH_2)_m-$ oder -Y-W-Gruppe, in der Y eine $-NR_4-$Gruppe oder auch, wenn A eine Bindung darstellt, ein Sauerstoffatom darstellt, wobei die vorstehenden Y- oder $NR_4$-Reste jeweils mit dem Rest A verknüpft sind sowie
m die Zahl 2, 3 oder 4,
n die Zahl 0 oder 1
$R_4$ ein Wasserstoffatom, eine Alkylgruppe mit 1 bis 3 Kohlenstoffatomen oder eine Benzylgruppe,
$R_5$ ein Wasserstoffatom oder
$R_4$ und $R_5$ sowie $R_4$ und $R_6$ zusammen jeweils eine Ethylengruppe und
W eine geradkettige Alkylengruppe mit 2 bis 5 Kohlenstoffatomen, wobei die Ethylengruppe in $\alpha$-Stellung durch eine Methoxyphenylethylaminocarbonylgruppe substituiert sein kann, oder eine 1,4-Cyclohexylengruppe darstellen,
E eine Alkoxycarbonylgruppe, in der der Alkoxyteil 2 bis 6 Kohlenstoffatome enthalten kann, eine Cycloalkoxycarbonylgruppe mit insgesamt 6 bis 10 Kohlenstoffatomen, in welcher der Cyclohexylteil zusätzlich durch 1 oder 2 Alkylgruppen mit jeweils 1 bis 3 Kohlenstoffatomen substituiert sein kann, eine Cycloalkoxycarbonylgruppe mit insgesamt 6 bis 8 Kohlenstoffatomen, in der im Cycloalkoxyteil eine Methylengruppe in 3- oder 4-Stellung durch ein Sauerstoffatom oder durch eine gegebenenfalls durch eine Methyl-, Benzyl-, Benzyloxycarbonyl- oder Acetylgruppe substituierte Iminogruppe ersetzt ist, eine Cycloalkylalkoxycarbonylgruppe, in der der Cycloalkylteil 5 oder 6 Kohlenstoffatome und der Alkoxyteil 1 oder 2 Kohlenstoffatome enthalten kann, eine Bicycloalkoxycarbonylgruppe mit insgesamt 8 Kohlenstoffatomen, eine $R_1$-CO-O-($R_2CR_3$)-O-CO-Gruppe, in der $R_1$ bis $R_3$ wie vorstehend erwähnt definiert sind, eine $R_7-CO-CH_2-O-CO-$Gruppe, in der $R_7$ eine Aminogruppe, die durch 1 oder 2 Methylgruppen oder durch eine Cyclohexylgruppe substituiert sein kann, eine Piperidinyl- oder Morpholinylgruppe darstellt, oder eine 1,3-Dihydro-3-oxo-1-isobenzfuranyloxycarbonylgruppe bedeuten,
deren Stereoisomere, einschließlich ihrer Gemische und deren Salze.

**4.** Biphenylderivate der allgemeinen Formel I gemäß Anspruch 1, in denen mit der Maßgabe, daß $R_a$ keine gegebenenfalls durch eine Alkoxycarbonylgruppe mit insgesamt 2 oder 3 Kohlenstoffatomen oder keine durch eine Benzyloxycarbonylgruppe substituierte Amidinogruppe darstellt, wenn E eine Alkoxycarbonylgruppe mit insgesamt 2 oder 3 Kohlenstoffatomen oder eine Benzyloxycarbonylgruppe darstellt,
$R_a$ eine gegebenenfalls durch eine $R_1$-CO-O-($R_2CR_3$)-O-CO-, Methoxycarbonyl- oder Allyloxycarbonylgruppe substituierte Amidinogruppe, wobei
$R_1$ eine geradkettige oder verzweigte Alkylgruppe mit 1 bis 4 Kohlenstoffatomen, eine Alkoxygruppe mit 1 bis 3 Kohlenstoffatomen oder eine Cyclohexyloxygruppe,
$R_2$ ein Wasserstoffatom oder eine Methylgruppe und

$R_3$ ein Wasserstoffatom darstellen,

$R_b$ ein Wasserstoffatom oder eine Methoxygruppe,

A eine $-CH_2-$ oder -CO-Gruppe und

B eine $-NR_4-CH_2CH_2-X-CH_2-$Gruppe, in der X eine $-NR_5-$Gruppe darstellt, eine $-NR_4-CH_2CH_2-R_6C=CH-$ oder -Y-W-Gruppe, in der Y eine $-NR_4-$Gruppe darstellt, wobei die vorstehenden Y- oder $NR_4$-Reste jeweils mit dem Rest A verknüpft sind sowie

$R_4$ ein Wasserstoffatom oder eine Methylgruppe oder

$R_4$ und $R_5$ sowie $R_4$ und $R_6$ zusammen jeweils eine Ethylengruppe und

W eine 1,4-Cyclohexylengruppe darstellen,

E eine Isopropyloxycarbonyl-, Isobutyloxycarbonyl-, 2-Butyloxycarbonyl-, 2-Amyloxycarbonyl-, 3-Amyloxycarbonyl- oder Neopentyloxycarbonylgruppe, eine Cycloalkoxycarbonylgruppe mit insgesamt 6 bis 9 Kohlenstoffatomen, in welcher der Cyclohexylteil zusätzlich durch 1 oder 2 Alkylgruppen mit jeweils 1 bis 3 Kohlenstoffatomen substituiert sein kann, eine Cycloalkylalkoxycarbonylgruppe, in der der Cycloalkylteil 5 oder 6 Kohlenstoffatome und der Alkoxyteil 1 oder 2 Kohlenstoffatome enthalten kann, eine Bicycloalkoxycarbonylgruppe mit insgesamt 8 Kohlenstoffatomen, eine $R_1-CO-O-(R_2CR_3)-O-CO$-Gruppe, in der $R_1$ bis $R_3$ wie vorstehend erwähnt definiert sind, bedeuten,

deren Stereoisomere, einschließlich ihrer Gemische und deren Salze.

**5.** Folgende Verbindungen der allgemeinen Formel I gemäß Anspruch 1:
4-Amidino-4'-[4-(cyclohexyloxycarbonylmethyl)-piperidinocarbonyl]-biphenyl,
4-Amidino-4'-[4-(cyclopentyloxycarbonylmethyl)-piperidinocarbonyl]-biphenyl,
4-Amidino-3'-[4-trans-(cyclohexyloxycarbonyl)-cyclohexylaminocarbonyl]-4'-methoxy-biphenyl,
4-Amidino-3'-[4-trans-(cyclohexylmethoxycarbonyl)-cyclohexylaminocarbonyl]-4'-methoxy-biphenyl,
4-Amidino-4'-[4-(cycloheptyloxycarbonylmethyl)-piperidinocarbonyl]-biphenyl,
4-Amidino-4'-[4-((exo)-norbornyloxycarbonylmethyl)-piperidinocarbonyl]-biphenyl,
4-Amidino-4'-[4-((-)-menthyloxycarbonylmethyl)-piperidinocarbonyl]-biphenyl,
4-Amidino-4'-[4-(pivaloyloxymethyloxycarbonylmethyl)-piperidinocarbonyl]-biphenyl,
4-[4-[(1-(Äthoxycarbonyloxy)-äthoxycarbonyl)-methyl]-piperidinocarbonyl]-4'-amidino-biphenyl,
[4-(N-Allyloxycarbonyl-amidino]-4'-[4-(methoxycarbonylmethyl)-piperidinocarbonyl]-biphenyl,
4-[N-Allyloxycarbonyl-amidino]-4'-[4-(carboxymethyl)-piperidinocarbonyl]-biphenyl und
4-[4-(Cyclohexyloxycarbonylmethyl)-piperidinocarbonyl]-4'-[N-methoxycarbonyl-amidino]-biphenyl,
deren Stereoisomere, einschließlich ihrer Gemische und deren Salze.

**6.** Physiologisch verträgliche Salze der Verbindungen nach mindestens einem der Ansprüche 1 bis 5 mit anorganischen oder organischen Säuren.

**7.** Arzneimittel, enthaltend eine Verbindung nach mindestens einem der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

**8.** Verwendung einer Verbindung nach mindestens einem der Ansprüche 1 bis 6 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

**9.** Verfahren zur Herstellung eines Arzneimittels gemäß Anspruch 7, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

**10.** Verfahren zur Herstellung der Biphenylderivate gemäß den Ansprüchen 1 bis 6, dadurch gekennzeichnet, daß

a) zur Herstellung von Verbindungen der allgemeinen Formel I, in der E eine Alkoxycarbonylgruppe mit insgesamt 3 bis 7 Kohlenstoffatomen, eine Phenylalkoxycarbonylgruppe, eine Cycloalkoxycarbonylgruppe mit insgesamt 4 bis 10 Kohlenstoffatomen, in welcher der Cycloalkylteil mit 5 bis 8 Kohlenstoffatomen zusätzlich durch 1 oder 2 Alkylgruppen substituiert sein kann, eine Cycloalkoxycarbonylgruppe mit insgesamt 6 bis 8 Kohlenstoffatomen, in der im Cycloalkoxyteil eine Methylengruppe in 3- oder 4-Stellung durch ein Sauerstoffatom oder durch eine gegebenenfalls durch eine Alkyl-, Phenylalkyl- oder Phenylalkoxycarbonylgruppe oder durch eine Alkanoylgruppe mit 2 bis 6 Kohlenstoffatomen substituierte Iminogruppe ersetzt ist und der Cycloalkoxyteil zusätzlich durch 1

oder 2 Alkylgruppen substituiert sein kann, eine Cycloalkenyloxycarbonylgruppe, in der der Cycloalkenylteil 5 bis 8 Kohlenstoffatome enthalten kann, eine Alkenyloxycarbonyl-, Phenylalkenyloxycarbonyl-, Alkinyloxycarbonyl- oder Phenylalkinyloxycarbonylgruppe mit der Maßgabe, daß keine Bindung an das Sauerstoffatom von einem Kohlenstoffatom ausgeht, welches eine Doppel- oder Dreifachbindung trägt und in denen der Alkenyl- und Alkinylteil jeweils 3 bis 5 Kohlenstoffatome enthalten kann, eine Cycloalkylalkoxycarbonylgruppe, in der der Cycloalkylteil 3 bis 8 Kohlenstoffatome enthalten kann, oder eine Bicycloalkoxycarbonylgruppe mit insgesamt 8 bis 10 Kohlenstoffatomen, die im Bicycloalkylteil zusätzlich durch ein oder zwei Alkylgruppen substituiert sein kann, darstellt, eine Carbonsäure der allgemeinen Formel

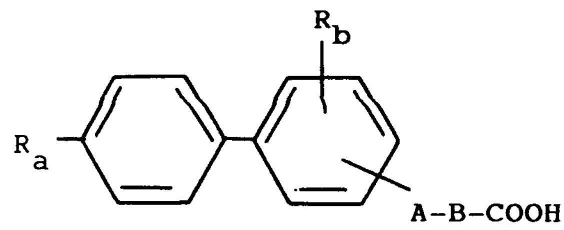

,(II)

in der

$R_a$, $R_b$, A und B wie in den Ansprüchen 1 bis 5 definiert sind, oder deren gegebenenfalls im Reaktionsgemisch gebildete reaktionsfähige Derivate, mit einem Alkohol der allgemeinen Formel

HO - $R_8$ ,(III)

in der

$R_8$ eine Alkylgruppe mit 2 bis 6 Kohlenstoffatomen, eine Phenylalkylgruppe, eine Cycloalkylgruppe mit 3 bis 9 Kohlenstoffatomen, in welcher der Cycloalkylteil mit 5 bis 8 Kohlenstoffatomen zusätzlich durch 1 oder 2 Alkylgruppen substituiert sein kann, eine Cycloalkylgruppe mit 5 bis 7 Kohlenstoffatomen, in der im Cycloalkylteil eine Methylengruppe in 3- oder 4-Stellung durch ein Sauerstoffatom oder durch eine gegebenenfalls durch eine Alkyl-, Phenylalkyl- oder Phenylalkoxycarbonylgruppe oder durch eine Alkanoylgruppe mit 2 bis 6 Kohlenstoffatomen substituierte Iminogruppe ersetzt ist und der Cycloalkylteil zusätzlich durch 1 oder 2 Alkylgruppen substituiert sein kann, eine Cycloalkenylgruppe, in der der Cycloalkenylteil 5 bis 8 Kohlenstoffatome enthalten kann, eine Alkenyl-, Phenylalkenyl-, Alkinyl- oder Phenylalkinylgruppe mit der Maßgabe, daß keine Bindung an das Sauerstoffmolekül von einem Kohlenstoffatom ausgeht, welches eine Doppel- oder Dreifachbindung trägt und in denen der Alkenyl- und Alkinylteil jeweils 3 bis 5 Kohlenstoffatome enthalten kann, eine Cycloalkylalkylgruppe, in der der Cycloalkylteil 3 bis 8 Kohlenstoffatome enthalten kann, oder eine Bicycloalkylgruppe mit insgesamt 7 bis 9 Kohlenstoffatomen, die im Bicycloalkylteil zusätzlich durch ein oder zwei Alkylgruppen substituiert sein kann, darstellt, verestert wird oder

b) eine Carbonsäure der allgemeinen Formel

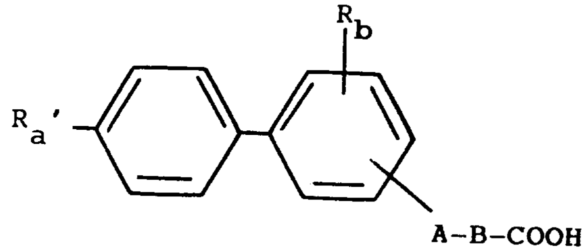

,(IV)

in der

$R_b$, A und B wie in den Ansprüchen 1 bis 5 definiert sind und

$R_a$' eine durch eine $R_1$-CO-O-($R_2CR_3$)-O-CO-, Alkoxycarbonyl-, Phenylalkoxycarbonyl-, Alkenyloxycarbonyl- oder Phenylalkenyloxycarbonylgruppe substituierte Amidinogruppe, wobei der vorstehend erwähnte Alkoxylteil jeweils 1 bis 6 Kohlenstoffatomen und der vorstehend erwähnte Alkenylteil 3 bis 5 Kohlenstoffatome enthalten kann, oder eine durch einen Schutzrest geschützte

Amidinogruppe darstellt, oder dessen Carbanion mit einer Verbindung der allgemeinen Formel

$Z_1$ - $R_9$ ,(V)

in der

$R_9$ eine Alkylgruppe mit 2 bis 6 Kohlenstoffatomen, eine Phenylalkylgruppe, eine Cycloalkylgruppe mit 3 bis 9 Kohlenstoffatomen, in welcher der Cycloalkylteil mit 5 bis 8 Kohlenstoffatomen zusätzlich durch 1 oder 2 Alkylgruppen substituiert sein kann, eine Cycloalkylgruppe mit 5 bis 7 Kohlenstoffatomen, in der im Cycloalkylteil eine Methylengruppe in 3- oder 4-Stellung durch ein Sauerstoffatom oder durch eine gegebenenfalls durch eine Alkyl-, Phenylalkyl- oder Phenyloxycarbonylgruppe oder durch eine Alkanoylgruppe mit 2 bis 6 Kohlenstoffatomen substituierte Iminogruppe ersetzt ist und der Cycloalkylteil zusätzlich durch 1 oder 2 Alkylgruppen substituiert sein kann, eine Cycloalkenylgruppe mit 5 bis 8 Kohlenstoffatomen, eine Alkenyl-, Phenylalkenyl-, Alkinyl- oder Phenylalkinylgruppe mit der Maßgabe, daß keine Bindung an $Z_1$ von einem Kohlenstoffatom ausgeht, welches eine Doppel- oder Dreifachbindung trägt und in denen der Alkenyl- und Alkinylteil jeweils 3 bis 5 Kohlenstoffatome enthalten kann, eine Cycloalkylalkylgruppe, in der der Cycloalkylteil 3 bis 8 Kohlenstoffatome enthalten kann, oder eine Bicycloalkylgruppe mit insgesamt 7 bis 9 Kohlenstoffatomen, die im Bicycloalkylteil zusätzlich durch ein oder zwei Alkylgruppen substituiert sein kann, eine $R_1$-CO-O-($R_2CR_3$)-Gruppe, in der $R_1$ bis $R_3$ wie in den Ansprüchen 1 bis 5 erwähnt definiert sind, eine $R_7$-CO-$CH_2$-Gruppe, in der $R_7$ eine Aminogruppe, die durch 1 oder 2 Alkylgruppen mit jeweils 1 bis 6 Kohlenstoffatomen oder durch eine Cycloalkylgruppe mit 3 bis 8 Kohlenstoffatomen substituiert sein kann, eine Pyrrolidinyl-, Piperidinyl-, Hexamethylenimino-, Morpholinyl- oder N-Alkylpiperazinyl-gruppe darstellt, oder eine 1,3-Dihydro-3-oxo-1-isobenzfuranylgruppe und

$Z_1$ eine nukleophile Austrittsgruppe bedeuten, umgesetzt wird und erforderlichenfalls anschließend ein gegebenenfalls verwendeter Schutzrest abgespalten wird oder

c) zur Herstellung von Verbindungen der allgemeinen Formel I, in der $R_a$ eine durch eine $R_1$-CO-O-($R_2CR_3$)-O-CO-, Alkoxycarbonyl-, Alkenyloxycarbonyl-, Phenylalkoxycarbonyl- oder Phenylalkenyloxycarbonylgruppe substituierte Amidinogruppe darstellt, eine Verbindung der allgemeinen Formel

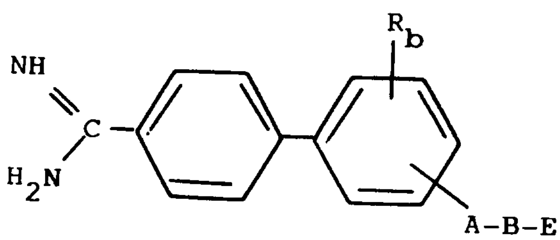

,(VI)

in der

$R_b$, A, B und E wie in den Ansprüchen 1 bis 5 definiert sind, mit einer Verbindung der allgemeinen Formel

$Z_2$ - $R_{10}$ ,(VII)

in der

$R_{10}$ eine $R_1$-CO-O-($R_2CR_3$)-O-CO-, Alkoxycarbonyl-, Alkenyloxycarbonyl-, Phenylalkoxycarbonyl- oder Phenylalkenyloxycarbonylgruppe und

$Z_2$ eine nukleophile Austrittsgruppe darstellen, acyliert wird und

erforderlichen falls ein während der Umsetzungen zum Schutz von reaktiven Gruppen verwendeter Schutzrest abgespalten wird und

gewünschtenfalls die so erhaltenen Verbindungen in ihre Enantiomeren und/oder Diastereomeren aufgetrennt werden und/oder

die erhaltenen Verbindungen der Formel I in ihre Salze, insbesondere für die pharmazeutische Anwendung in ihre physiologisch verträglichen Salze mit anorganischen oder organischen Säuren, übergeführt werden.

Europäisches Patentamt

# EUROPÄISCHER RECHERCHENBERICHT

Nummer der Anmeldung

EP 93 10 9190

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,X | EP-A-0 496 378 (DR. KARL THOMAE GMBH) 29. Juli 1992 Beispiele 6 (72) und 8 (1)-(4) * Anspruch 1 * --- | 1-10 | C07D211/32 A61K31/445 A61K31/495 C07D295/10 |
| P,A | EP-A-0 518 819 (CIBA GEIGY) 16. Dezember 1992 * Anspruch 1 * --- | 1-10 | |
| P,A | EP-A-0 528 369 (DR. KARL THOMAE GMBH) 24. Februar 1993 * Anspruch 1 * --- | 1-10 | |
| A | EP-A-0 381 033 (F.HOFFMANN-LA ROCHE AG) 8. August 1990 * Anspruch 1 * --- | 1-10 | |
| A | EP-A-0 483 667 (DR. KARL THOMAE GMBH) 6. Mai 1992 * Anspruch 1 * ----- | 1-10 | |
| | | | RECHERCHIERTE SACHGEBIETE (Int. Cl.5) |
| | | | C07D A61K |

Der vorliegende Recherchenbericht wurde für alle Patentansprüche erstellt

| Recherchenort | Abschlußdatum der Recherche | Prüfer |
|---|---|---|
| MUENCHEN | 27 SEPTEMBER 1993 | GETTINS M.P. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)